# FASCICULE DE BREVET EUROPEEN

(11) **EP 1 250 417 B1**
(45) Date de publication et mention de la délivrance du brevet: **24.03.2004**
(21) Numéro de dépôt: 01903953.6
(22) Date de dépôt: 15.01.2001
(51) Int. Cl.: C12M 3/06, C12M 1/00

(54) **DISPOSITIF DE CULTURE CELLULAIRE ET TISSULAIRE A CIRCULATION DE FLUIDE DE CULTURE CONTROLEE**
ZELL- UND GEWEBEKULTURVORRICHTUNG MIT GESTEURTE FLUIDUMZIRKULATION
CELL AND TISSUE CULTURE DEVICE WITH CONTROLLED CULTURE FLUID CIRCULATION

(30) Priorité: 17.01.2000 FR 0000547
(43) Date de publication de la demande: 23.10.2002
(73) Titulaire: Sarem, Farzin, 54500 Vandoeuvre les Nancy (FR)
(72) Inventeur: SAREM, Farzin, F-06560 Valbonne (FR); SAREM DAMERDJI, Leila-Ouassila, F-06560 Valbonne (FR)
(74) Mandataire: Nithardt, Roland
(86) Numéro de dépôt international: PCT/FR2001/000122
(87) Numéro de publication internationale: WO 2001/053451

(56) Documents cités:
- WO-A-96/12789
- US-A- 5 707 868

## Description

L'invention concerne un dispositif et un procédé de culture cellulaire et tissulaire à circulation de fluide de culture contrôlée.

Cette invention concerne plus particulièrement les dispositifs et procédés de culture de cellules et tissus dans lesquels le fluide de culture (ou nutriment) est mis en mouvement de manière à permettre une culture dynamique. De tels dispositifs mettent en oeuvre, habituellement, une technique d'agitation du fluide de culture directement dans l'espace de culture ou bien une technique de circulation permanente du fluide à l'aide d'un circuit spécifique.

Ces techniques de circulation de fluide sont difficiles à maîtriser en raison de l'évolution des conditions de culture au cours du temps. En effet, la faible concentration cellulaire qui existe au début de la culture, requiert un environnement quasi-statique, et par conséquent un débit de fluide de culture très faible, alors que la multiplication des cellules ou la croissance des tissus nécessite un débit croissant rapidement.

Le maintien des conditions adéquates pendant toute la durée de la culture requiert soit l'utilisation de plusieurs dispositifs appropriés, respectivement, aux différents stades d'évolution de la culture, ce qui impose de nombreuses manipulations, soit des moyens complexes et souvent encombrants, qui limitent, voire interdisent, l'observation de l'évolution de la culture à l'aide d'un microscope. Ces manipulations et moyens sont d'autant plus complexes que la culture doit s'effectuer dans un environnement stérile qui requiert du personnel hautement qualifié, et par conséquent accroît le coût de chaque culture. De plus, du fait de leur coût, ces dispositifs doivent être réutilisables, ce qui impose une stérilisation complète avant chaque nouvelle culture et des opérations de maintenance.

On connaît par la publication WO 96/12789 une installation de fermentation comportant une enceinte de volume fixe à l'intérieur de laquelle est logée une chambre de culture, de volume variable, raccordée à deux réservoirs. Seule la gestion de l'alimentation de la chambre de culture permet d'en faire varier le volume.

Dans le dispositif de culture objet du brevet US 5,707,868, la variation du volume de la chambre de culture est obtenue en déplaçant une paroi mobile de cette chambre.

De par leur conception, ces dispositifs ne permettent pas d'obtenir un bon contrôle de l'évolution de la culture.

L'invention a donc pour but de résoudre tout ou partie des inconvénients précités.

Elle propose, à cet effet, un dispositif tel que défini en préambule et caractérisé en ce qu'il comprend :
- un circuit de pressurisation agencé pour délivrer au moins un gaz choisi, sous une pression choisie,
- au moins une chambre de culture logeant des moyens propres à supporter au moins une membrane déformable formant dans la chambre une interface entre de première et seconde parties à volumes complémentaires variables, ladite première partie étant agencée pour recevoir des cellules et/ou des tissus à cultiver et pour être alimentée en fluide de culture par un premier réservoir, et ladite seconde partie comprenant une entrée propre à être alimentée en gaz par ledit circuit de pressurisation et une sortie pour évacuer une partie au moins de ce gaz, et
- des moyens de commande agencés pour déterminer, en fonction de critères choisis relatifs au type de culture à effectuer, le gaz à introduire dans la seconde partie, sa pression et la durée d'introduction de ce gaz, et pour contrôler les accès aux première et seconde parties de la chambre de manière à gérer conjointement la forme de la membrane, l'alimentation en fluide de culture et la circulation de ce fluide dans la première partie.

Le fait de faire varier la pression du gaz dans la seconde partie, permet de modifier la forme de la ou des membranes, et par conséquent de contraindre le fluide de culture à circuler dans la première partie de la chambre. Par exemple, on peut alterner des phases de surpression avec des phases de dépression dans la seconde partie de la chambre pour provoquer l'oscillation de la ou des membranes.

Ainsi, il suffit, par exemple, de définir pour chaque type de culture des suites de multiplets de paramètres comportant chacun au moins un gaz choisi, une pression pour ce gaz, une durée d'alimentation en ce gaz, un débit de fluide de culture et une durée d'alimentation en ce fluide, pour contrôler l'évolution de la culture. Mais, on peut envisager des modes de fonctionnement plus complexes, reposant sur une adaptation des paramètres des multiplets à partir de mesures physiques, telles que des mesures de température, ou de proportion d'espèce(s) de molécules, ou encore de pH, effectuées dans la chambre et/ou dans le circuit de pressurisation.

Tous ces paramètres peuvent être mémorisés et/ou calculés dans les moyens de commande. Ils peuvent également être modifiés par une reprogrammation.
On peut également envisager de faire des mesures de pression dans la chambre de culture, ou des mesures de niveau de fluide de culture dans la chambre de culture, ou encore des mesures colorimétriques pour quantifier l'évolution de certaines cultures, ou encore des mesures de potentiel redox.

Dans le cas d'un dispositif à une seule membrane, ce dispositif peut comporter une sur-membrane placée au-dessus de la membrane, du côté de la première partie et délimitant avec cette membrane au moins une zone intermédiaire, ladite chambre comprenant au moins une entrée propre à être alimentée en gaz par le circuit de pressurisation et à alimenter en ledit gaz ladite zone intermédiaire et au moins une sortie pour évacuer de cette zone intermédiaire une partie au moins du gaz, et lesdits moyens de commande étant agencés pour déterminer, en fonction des critères choisis, le gaz à introduire dans la zone intermédiaire, sa pression et la durée d'introduction de ce gaz, et pour contrôler les accès aux entrée et sortie de cette zone intermédiaire de manière à gérer conjointement les formes de la membrane et de la sur-membrane et la circulation du fluide de culture dans la première partie.

La sur-membrane peut être solidarisée à la membrane en une multiplicité d'endroits choisis, de manière à subdiviser la zone intermédiaire en une pluralité d'alvéoles communicantes à volumes variables.

De façon avantageuse, ledit circuit de pressurisation comporte au moins une entrée raccordée à un réservoir contenant ledit gaz choisi et au moins une sortie alimentant l'entrée de la seconde partie.

Dans la forme de réalisation préférée, ladite première partie comprend au moins une entrée propre à être alimentée en fluide de culture par ledit premier réservoir et au moins une sortie propre à évacuer le fluide de culture usagé dans un second réservoir, et lesdits moyens de commande sont agencés pour contrôler les accès aux différentes entrées et sorties des première et seconde parties de manière à gérer conjointement la forme de la membrane, l'alimentation en fluide de culture et la circulation de ce fluide dans ta première partie.

Ledit circuit de pressurisation peut être de type fermé et peut comporter au moins une entrée propre à l'alimenter en gaz choisi, un compresseur propre à délivrer le gaz choisi sous la pression choisie et au moins une sortie alimentée par ledit compresseur et propre à alimenter l'entrée de la seconde partie.

De façon avantageuse, lesdits moyens de support sont agencés pour supporter une multiplicité de membranes indépendantes. Ainsi, selon le type de programmation des moyens de commande, et selon le nombre de groupes de membranes, il est possible de générer de nombreux modes de circulation de fluide dans la première partie. On citera à titre d'exemple une circulation essentiellement «horizontale», une circulation essentiellement «verticale», une combinaison de circulations verticale et horizontale, ou bien encore une circulation en «zigzag».

Dans une première forme de réalisation, ladite multiplicité est subdivisée en au moins deux groupes de membranes indépendants, et ladite seconde partie comporte au moins deux entrées pour alimenter en gaz choisi chaque groupe de membranes, indépendamment, lesdits moyens de commande étant agencés pour contrôler l'accès à ces deux entrées de manière à gérer les formes respectives des membranes de chaque groupe.

Selon une seconde forme de réalisation, ladite multiplicité peut être subdivisée en quatre groupes de membranes indépendantes, ladite seconde partie comportant quatre entrées pour alimenter en gaz choisi chaque groupe de membranes, indépendamment, et lesdits moyens de commande étant agencés pour contrôler l'accès à ces quatre entrées de manière à gérer les formes respectives des membranes de chaque groupe.

Ladite chambre de culture peut loger dans la première partie, du côté opposé à la seconde partie, une poche déformable munie d'une entrée alimentée en. gaz par le circuit de pressurisation et d'une sortie raccordée à ce circuit de pressurisation, et lesdits moyens de commande sont agencés pour déterminer, en fonction desdits critères relatifs au type de culture à effectuer, la pression et la durée d'introduction de ce gaz, et pour contrôler les accès à l'entrée et à la sortie de la poche de manière à gérer sa forme conjointement avec la forme de chaque membrane. De préférence, ladite poche est formée d'une membrane.

Le nombre de modes de circulation peut être encore accru si l'on munit la première partie de la chambre de culture, du côté opposé à la seconde partie, de moyens de support auxiliaires propres à supporter au moins une membrane auxiliaire déformable formant dans la première partie une interface entre une partie centrale propre à recevoir les cellules ou tissus à cultiver et une partie arrière comportant une entrée propre à être alimentée en gaz par le circuit de pressurisation et une sortie raccordée à ce circuit de pressurisation, lesdits moyens de commande étant agencés pour déterminer, en fonction desdits critères relatifs au type de culture à effectuer, la pression et la durée d'introduction de ce gaz, et pour contrôler les accès à l'entrée et à la sortie de la partie arrière de manière à gérer la forme de la membrane auxiliaire conjointement avec la forme de chaque membrane.

De préférence, ledit circuit de pressurisation comporte une autre sortie propre à alimenter en ledit gaz choisi, sous une autre pression choisie, l'entrée de la poche ou de la partie arrière. L'entrée et la sortie de la poche ou de la partie arrière sont de préférence confondues.

Lesdits moyens de support auxiliaire sont agencés pour supporter une multiplicité de membranes auxiliaires indépendantes.

Ladite multiplicité peut être subdivisée en au moins deux groupes de membranes auxiliaires indépendants, ladite partie arrière comportant au moins deux entrées pour alimenter en gaz choisi chaque groupe de membranes auxiliaires, indépendamment, et lesdits moyens de commande étant agencés pour contrôler l'accès à ces deux entrées de manière à gérer les formes respectives des membranes auxiliaires de chaque groupe.

Ladite multiplicité peut également être subdivisée en quatre groupes de membranes auxiliaires indépendantes, ladite partie arrière comportant quatre entrées pour alimenter en gaz choisi chaque groupe de membranes auxiliaires, indépendamment, et lesdits moyens de commande étant agencés pour contrôler l'accès à ces quatre entrées de manière à gérer les formes respectives des membranes auxiliaires de chaque groupe.

De façon avantageuse, la partie centrale de la première partie loge des moyens de cloisonnement définissant dans celle-ci des cavités de culture s'étendant chacune d'un groupe de membranes à un groupe de membranes auxiliaires, chaque cavité étant propre à être alimentée en fluide de culture. Lesdits moyens de cloisonnement comportent de préférence des cloisons réalisées dans un matériau poreux audit fluide de culture, mais imperméable aux cellules et tissus, de sorte que ledit fluide de culture puisse circuler d'une cavité à l'autre.

Dans toutes les variantes de réalisation, la première partie comporte au moins une ouverture munie d'un moyen étanche, en particulier de type septum, propre à permettre l'introduction ou l'extraction de cellule ou tissus et l'entrée et la sortie de la seconde partie sont confondues.

Lesdites membranes et membranes auxiliaires sont avantageusement réalisées dans un matériau poreux, au moins dans le sens allant vers la première partie, de sorte que le gaz choisi, introduit dans la seconde partie, puisse pénétrer au moins partiellement dans ladite première partie.

Ladite chambre de culture est délimitée par un boîtier comprenant une partie inférieure formant réceptacle, comportant au moins une ouverture adaptée pour l'entrée d'alimentation en fluide de culture, la sortie d'évacuation du fluide de culture usé, l'entrée (ou les entrées) d'alimentation en gaz choisi de la seconde partie et la (ou les) sortie(s) raccordée(s) au circuit de pressurisation, une partie supérieure formant couvercle, lesdits moyens de support comprenant un bâti logé dans le réceptacle et dans lequel se trouvent définies des cavités propres chacune à supporter à étanchéité une membrane, à être alimentée en ledit gaz choisi et à loger ladite membrane lorsque la pression du gaz choisi est inférieure à un premier seuil.

De préférence, ledit couvercle est réalisé dans un matériau transparent.

De façon avantageuse, ledit réceptacle comporte une ouverture pour chaque entrée et pour chaque sortie des première et seconde parties de la chambre.

Dans une variante, ledit réceptacle comporte une ouverture pour chaque entrée et pour chaque sortie de la seconde partie de la chambre, ledit couvercle comprend une ouverture adaptée à l'entrée et à la sortie de la poche ou de la partie arrière, et lesdits moyens de support auxiliaire sont solidarisés audit couvercle.

Le dispositif peut comprendre au moins une sonde, choisie parmi au moins une sonde de pH et une sonde de température, raccordée auxdits moyens de commande et propre à être introduite à l'intérieur de la première partie de la chambre, et ledit réceptacle comporte au moins une première ouverture auxiliaire adaptée à l'introduction de chaque sonde.

Le dispositif peut également comprendre deux entrées pilotées par lesdits moyens de commande et propres à être alimentées respectivement en de premier et second gaz choisis.

Dans toutes les réalisations, chaque entrée et chaque sortie sont contrôlées par un moyen de commande d'accès piloté par lesdits moyens de commande.

Ledit bâti est de préférence réalisé dans un matériau synthétique, en particulier en élastomère.

Ladite première partie loge au moins une membrane latérale déformable formant dans la première partie, une interface entre une partie latérale et la partie contenant les cellules ou tissus, ladite partie latérale comprenant une entrée propre à être alimentée en gaz par ledit circuit de pressurisation et une sortie pour évacuer une partie au moins de ce gaz, et les moyens de commande sont agencés pour déterminer, en fonction des critères choisis, la pression et la durée d'introduction du gaz, et pour contrôler les accès aux entrée et sortie de la partie latérale de manière à gérer la forme de la membrane latérale. Le gaz introduit dans ladite partie latérale est le gaz choisi.

Dans toutes les variantes de réalisation, le dispositif peut comprendre au moins une unité de culture comportant le premier réservoir raccordé à la chambre de culture et un second réservoir raccordé à cette chambre de culture de manière à recueillir le fluide de culture usé. Chaque premier et second réservoir comprend au moins une ouverture munie d'un moyen étanche, en particulier de type septum, propre à permettre l'introduction ou l'extraction de fluide.

L'un au moins des premier et second réservoir comprend au moins une poche-réservoir souple propre à alimenter en fluide de culture ladite première partie ou à recueillir le fluide usagé.

De façon avantageuse, au moins le premier réservoir comprend au moins deux poches-réservoir souples propre à alimenter en fluide de culture différents ladite première partie.

Le dispositif comprend en outre un premier compartiment logeant les moyens de commande et le circuit de pressurisation, et un second compartiment logeant ladite unité de culture. Ledit second compartiment peut également loger au moins une autre unité de culture, et ledit circuit de pressurisation comprend au moins une première partie de dérivation, raccordée à chacune de ses sorties délivrant ledit gaz sous pression choisie, de manière à alimenter en gaz au moins l'autre unité de culture.

Ledit circuit de pressurisation comprend avantageusement une seconde partie de dérivation, raccordée à chacune de ses sorties délivrant ledit gaz sous pression choisie, de manière à alimenter en gaz au moins une unité de culture externe.

Ledit premier compartiment comprend un sous-compartiment formant réservoir de gaz alimenté par chaque entrée d'alimentation en gaz choisi et alimentant ledit compresseur. Ledit sous-compartiment loge un capteur de température agencé pour délivrer des mesures de température aux moyens de commande et un moyen de chauffage du gaz piloté par lesdits moyens de contrôle en fonction desdits critères choisis et desdites mesures de température. Il loge également un analyseur de gaz agencé pour délivrer aux moyens de commande des mesures représentatives du pourcentage d'au moins une espèce de molécules à l'intérieur dudit sous-compartiment, et lesdits moyens de commande sont agencés pour contrôler l'accès aux différentes entrées et sorties en fonction desdits critères choisis et desdites mesures de pourcentage.

Ledit circuit de pressurisation comporte avantageusement un conduit reliant au moins une sortie dudit sous-compartiment à l'entrée de ladite poche ou de ladite partie arrière de la première partie.

Ledit sous-compartiment est de préférence subdivisé en au moins une première et une seconde sous-partie étanches formant respectivement un premier réservoir de gaz basse pression, alimenté par chaque entrée d'alimentation en gaz choisi et alimentant ledit compresseur, et un second réservoir de gaz haute pression alimenté par ledit compresseur et alimentant chaque sortie du circuit de pressurisation en gaz choisi. La première sous-partie du sous-compartiment loge ledit analyseur de gaz et la seconde sous-partie du sous-compartiment loge ledit capteur de température et ledit moyen de chauffage du gaz.

Dans une variante de réalisation, ledit sous-compartiment est subdivisé en une première, une seconde une troisième sous-partie étanches, ladite troisième sous-partie étant placée entre les première et seconde sous-parties et formant un troisième réservoir de gaz de pression intermédiaire, lesdites sous-parties communicant entre elles par une ouverture à accès contrôlé par lesdits moyens de commande. Chaque sous-partie alimente en gaz choisi, sous contrôle de moyens de contrôle d'accès pilotés par lesdits moyens de commande, chaque sortie du circuit de pressurisation, délivrant ledit gaz choisi à ladite chambre de culture.

L'invention concerne également un procédé tel que défini en préambule et
caractérisé en ce qu'il comprend :
- une première étape dans laquelle on prévoit un dispositif de culture comprenant au moins une chambre de culture munie d'au moins une membrane déformable à l'interface entre de première et seconde parties à volumes complémentaires variables, ladite première partie étant propre à être alimentée en un fluide de culture et à recevoir des cellules et/ou des tissus à cultiver, et ladite seconde partie étant propre à être alimentée en un gaz ; et
- une seconde étape dans laquelle on alimente lesdites première et seconde parties selon des pressions, débits et durées choisis en fonction de critères relatifs au type de culture à effectuer, pour faire varier la forme de cette membrane pendant la durée de la culture, de manière à créer une circulation contrôlée du fluide de culture dans la première partie de la chambre.

De façon avantageuse, à la première étape on prévoit une chambre comprenant une multiplicité de membranes indépendantes ainsi qu'une subdivision de la multiplicité en au moins deux groupes de membranes indépendants, et à la seconde étape on alimente indépendamment en gaz choisi chaque groupe, de manière à faire varier indépendamment les formes respectives des membranes de chaque groupe.

A la première étape on peut également prévoit une subdivision de la multiplicité en au moins quatre groupes de membranes indépendants, et à la seconde étape on peut alimenter indépendamment en gaz choisi chaque groupe, de manière à faire varier indépendamment les formes respectives des membranes de chaque groupe

Dans une première variante du procédé, à la première étape on prévoit dans la première partie de la chambre de culture, du côté opposé à la seconde partie, une poche déformable alimentée en gaz, et à la seconde étape on alimente ladite poche selon des pressions, débits et durées choisis en fonction desdits critères, pour faire varier la forme de cette poche conjointement avec celle de chaque membrane pendant la durée de la culture, de manière à créer la circulation du fluide de culture.

Dans une seconde variante, à la première étape on prévoit dans la première partie de la chambre de culture, du côté opposé à la seconde partie, au moins une membrane auxiliaire déformable formant interface entre une partie centrale recevant les cellules ou tissus à cultiver et une partie arrière alimentée en gaz, et à la seconde étape on alimente ladite partie arrière selon des pressions, débits et durées choisis en fonction desdits critères, pour faire varier la forme de la membrane auxiliaire conjointement avec celle de chaque membrane pendant la durée de la culture, de manière à créer la circulation de fluide de culture.

A la première étape la seconde partie et la poche ou la partie arrière sont alimentées en le même gaz choisi.

De préférence, à la première étape, chaque membrane et la poche ou la membrane auxiliaire sont réalisées dans un matériaux poreux, au moins dans le sens allant vers la première partie, de sorte que le gaz choisi, introduit dans la seconde partie et/ou la poche ou la membrane auxiliaire, puisse pénétrer au moins partiellement dans ladite première partie.

La seconde étape est propre à assurer un déplacement du fluide et/ou des cellules et tissus de manière à créer dans la chambre de culture des zones dans lesquelles les concentrations cellulaires sont différentes entre elles et, dans cette étape, les différences de concentrations cellulaires s'effectuent par des modifications locales du volume de culture de la première partie et notamment par des concentrations cellulaires initiales différentes dans lesdites zones de culture et/ou des cycles de déformation de membrane(s) et/ou poche qui diffèrent d'une zone à l'autre.

La seconde étape est également propre à assurer conjointement un contrôle de la pression et de la circulation du fluide de culture dans la première partie de la chambre.

A cet effet, dans la première étape on prévoit dans la première partie des moyens de cloisonnement définissant des cavités de culture s'étendant chacune d'un groupe de membranes à un groupe de membranes auxiliaires, et propres chacune à être alimentées en fluide de culture et à recevoir des cellules adhérentes ainsi que des supports de culture et/ou des cellules non adhérentes et/ou des tissus, éventuellement différents d'une cavité à l'autre.

Ainsi, comme indiqué pour le dispositif, selon les pressions, durées et débits appliqués, et selon le nombre de groupes de membranes utilisés, il est possible de générer de nombreux modes de circulation de fluide dans la première partie.

D'autres caractéristiques et avantages de l'invention apparaîtront à l'examen de la description détaillée ci-après, et des dessins annexés, sur lesquels :
- la figure 1 illustre de façon schématique un premier mode de réalisation d'un dispositif de culture selon l'invention,
- la figure 2 illustre de façon détaillée la chambre de culture du dispositif de la figure 1,
- les figures 3A à 3C illustrent schématiquement, dans des vues en coupe transversale, trois états différents de la membrane inférieure contenue dans la chambre de la figure 2, qui permettent de faire circuler le fluide de culture,
- les figures 4A et 4B illustrent schématiquement, dans des vues en coupe transversale, deux états différents de la membrane inférieure contenue dans la chambre de la figure 2, qui permettent d'évacuer le fluide de culture usé,
- les figures 5A et 5B illustrent schématiquement, dans des vues en coupe transversale, une variante du dispositif de la figure 2, dans deux états différents de la sur-membrane placée au-dessus de la membrane inférieure,
- la figure 6 illustre de façon détaillée une variante de chambre de culture, à deux membranes inférieure et supérieure,
- les figures 7A à 7C illustrent schématiquement, dans des vues en coupe transversale, trois états différents des membranes inférieure et supérieure contenues dans la chambre de la figure 6, qui permettent de faire circuler le fluide de culture,
- les figures 8A et 8B illustrent schématiquement, dans des vues en coupe transversale, deux états différents des membranes inférieure et supérieure contenues dans la chambre de la figure 6, qui permettent d'évacuer le fluide de culture usé,
- la figure 9 illustre, dans une vue éclatée, une autre variante de chambre de culture du dispositif de la figure 1, à membranes inférieures multiples et poche supérieure déformable,
- les figures 10A et 10B illustrent schématiquement, dans des vues en coupe transversale, deux états différents des membranes inférieures et de la poche supérieure contenues dans la chambre de la figure 9, qui permettent de faire circuler le fluide de culture,
- les figures 11A et 11B illustrent schématiquement, dans des vues du dessus, déployé, et de côté, une unité de culture comportant une chambre du type de celles illustrées sur les figures 9 et 10,
- la figure 12 illustre une variante du dispositif de la figure 1, à deux unités de culture,
- les figures 13A et 13B illustrent une autre variante de dispositif selon l'invention, à triple réservoir de gaz,
- les figures 14A à 14C illustrent schématiquement trois états différents des membranes inférieures multiples de la chambre de culture de la figure 9,
- les figures 15A et 15B illustrent schématiquement deux états différents des membranes inférieures multiples et de la poche supérieure de la chambre de culture de la figure 9, dans une culture de type monophase,
- les figures 16A et 16B illustrent schématiquement deux états différents des membranes inférieures multiples et de la poche supérieure de la chambre de culture de la figure 9, dans une culture de type biphase,
- les figures 17A et 17B illustrent schématiquement, dans des vues du dessus et en coupe transversale, deux états successifs des membranes inférieures multiples et de la poche supérieure de la chambre de culture de la figure 9, dans un premier exemple de mode de circulation de fluide de culture,
- les figures 18A et 18B illustrent schématiquement, dans des vues du dessus et en coupe transversale, deux états successifs des membranes inférieures multiples et de la poche supérieure de la chambre de culture de la figure 9, dans un second exemple de mode de circulation de fluide de culture,
- les figures 19A à 19D illustrent schématiquement, dans des vues du dessus et en coupe transversale, quatre états successifs des membranes inférieures multiples et de la poche supérieure de la chambre de culture de la figure 9, dans un troisième exemple de mode de circulation de fluide de culture,
- les figures 20A et 20B illustrent schématiquement, dans des vues du dessus et en coupe transversale, deux états successifs des membranes inférieures multiples et de la poche supérieure de la chambre de culture de la figure 9, dans un quatrième exemple de mode de circulation de fluide de culture,
- les figures 21A à 21D illustrent schématiquement, dans des vues du dessus et en coupe transversale, quatre états successifs des membranes inférieures multiples et de la poche supérieure de la chambre de culture de la figure 9, dans un cinquième exemple de mode de circulation de fluide de culture,
- les figures 22A et 22B illustrent schématiquement, dans des vues du dessus et en coupe transversale, deux états successifs des membranes inférieures multiples et de la poche supérieure de la chambre de culture de la figure 9, dans un exemple de mode d'évacuation du fluide de culture usé, et
- les figures 23A et 23B illustrent schématiquement, dans des vues du dessus et en coupe transversale, deux états successifs des membranes inférieures multiples et de la poche supérieure de la chambre de culture de la figure 9, dans un exemple de mode d'alimentation en fluide de culture.

Les dessins annexés sont, pour l'essentiel, de caractère certain. En conséquence, ils pourront non seulement servir à compléter l'invention, mais aussi contribuer à sa définition, le cas échéant.

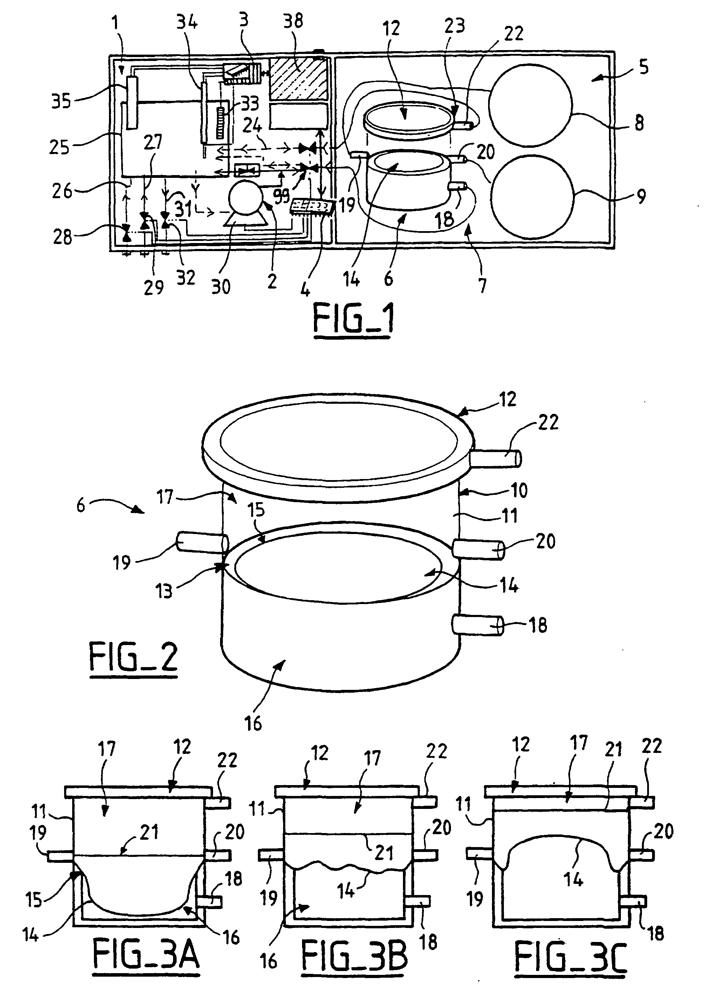

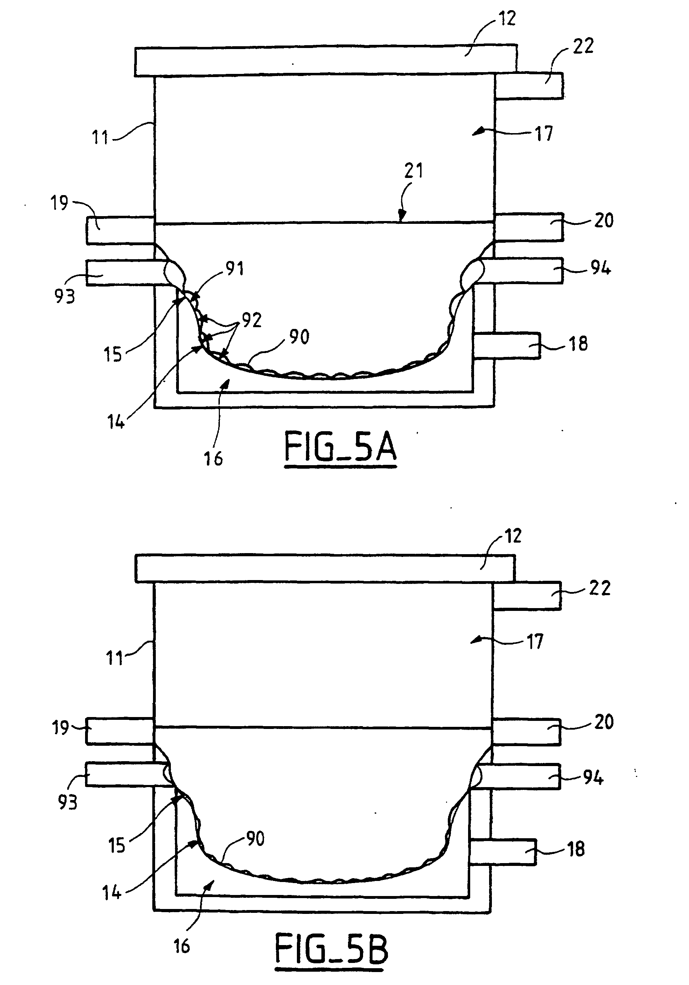

On se réfère tout d'abord à la figure 1 pour décrire un premier dispositif de culture de cellules et tissus, dans un mode de réalisation non limitatif. Le dispositif illustré par cette figure comporte un premier compartiment dans lequel se trouve logés un circuit de pressurisation 2, qui sera décrit plus loin, et des moyens de commande 3, 4, destinés à piloter le dispositif et qui seront également décrits plus loin. Ledit dispositif comporte également un second compartiment 5 dans lequel se trouve logée une unité de culture 6 comportant une chambre de culture 7 raccordée à un premier réservoir 8 d'alimentation en fluide de culture, ainsi qu'à un second réservoir 9 de collection de fluide de culture usé. Le circuit de pressurisation 2 est raccordé à l'unité de culture 6, de manière à gérer son alimentation en fluide de culture, ainsi que la circulation dudit fluide de culture à l'intérieur de la chambre 7, comme cela sera décrit ci-après.

Comme cela est mieux illustré sur la figure 2, la chambre de culture 6 peut être réalisée sous la forme d'un boîtier 10 comportant une partie, que l'on qualifiera "d'inférieure", formant réceptacle 11, et une autre partie, que l'on qualifiera de "supérieure", formant couvercle 12 propre à obturer, de façon étanche, la partie supérieure ouverte du réceptacle 11. Le couvercle 12 est de préférence amovible pour permettre l'introduction des cellules et tissus à cultiver ainsi qu'éventuellement un ou plusieurs supports de culture. Par ailleurs, ce couvercle est de préférence transparent pour permettre l'observation de l'évolution de la culture.

Le réceptacle 11 (partie inférieure du boîtier 10) loge des moyens de support 13 permettant de maintenir, à une altitude choisie par rapport au fond du réceptacle 11, le bord périphérique d'une membrane 14. Selon l'invention, cette membrane est réalisée dans un matériau déformable tel que le silicone, ou le polydimethylsiloxane (PDSM), ou encore le polytetrafluorethylène (PTFE), ou encore les polymères dimethyl et methylvinyl siloxanes. Elle forme dans la chambre de culture une interface entre une première partie supérieure 17 (ci-après partie supérieure 17) et une seconde partie inférieure 16 (ci-après partie inférieure 16) à volumes complémentaires variables.

La déformation de la membrane 14 s'effectue par une gestion de pressions, débits et durées d'alimentation, à l'aide du circuit de pressurisation 2, et sous contrôle des moyens de commande 3 et 4. Pour ce faire, le réceptacle 11 comporte, dans la partie supérieure 17, une première entrée 18 raccordée à une branche principale 99 du circuit de pressurisation 2. De préférence, la première entrée 18 est en fait une entrée/sortie permettant l'introduction d'un gaz, sous une pression choisie, à l'intérieur de la partie inférieure 16, tout comme l'extraction d'une partie au moins de ce gaz.

Ainsi, comme illustré sur les figures 3A à 3C, selon la pression du gaz qui est introduit, via l'entrée/sortie 18, à l'intérieur de la partie inférieure 16, la membrane est soit logée à l'intérieur de la partie inférieure 16 (figure 3A), soit à l'intérieur de la partie supérieure 17 (figure 3C), soit à un niveau intermédiaire entre les deux cas précités (figure 3B). Plus précisément, l'extraction du gaz hors de la partie inférieure 16 génère une dépression qui contraint la membrane 14 à se rapprocher du fond du réceptacle 11 (figure 3A). En revanche, l'introduction d'un gaz sous une pression choisie (haute pression) à l'intérieur de cette partie inférieure 16, via l'entrée 18, repousse la membrane 14 vers la partie supérieure 17 du réceptacle 11 (comme illustré sur les figures 3B et 3C). Avantageusement, le réceptacle 11 comporte, dans la partie supérieure 17, au-dessus de la membrane 14, une seconde entrée 19 raccordée au réservoir d'alimentation 8, ainsi qu'une sortie 20 raccordée au réservoir de collection 9. Les accès aux différentes entrées et sorties 18, 19 et 20 sont contrôlés par les moyens de commande 3 et 4, de préférence grâce à des électrovannes. Ces moyens de commande, une fois programmés, fournissent aux cellules ou tissus qui sont préalablement introduits à l'intérieur de la partie supérieure 17 de la chambre 6, au-dessus de la membrane 14, un flux de fluide de culture dont les paramètres de pression, débit, et durée d'introduction, sont, à chaque instant de la culture, adaptés aux besoins de celles-ci. La circulation du fluide de culture à l'intérieur de la partie supérieure 17 est contrôlée par le déplacement de la membrane 14, dû aux variations de pression, débit et durée d'introduction du gaz dans la partie inférieure 16.

Sur les figures 3A à 3C, se trouve matérialisé par un trait horizontal 21 le niveau du fluide de culture à l'intérieur de la chambre 6. Au fur et à mesure que la pression du gaz augmente à l'intérieur de la partie inférieure 16, le niveau 21 monte à l'intérieur de la partie supérieure 17, en direction du couvercle 12. Du fait de la génération de gaz à l'intérieur de la partie supérieure 17, il est préférable de prévoir dans celle-ci une entrée/sortie 22 permettant l'extraction du gaz résiduel. Cette entrée/sortie 22 est de préférence à accès contrôlé par les moyens de commande 3 et 4, mais il pourrait également s'agir de moyens d'accès contrôlés manuellement par un opérateur. Par ailleurs, comme illustré sur la figure 2, cette entrée/sortie 22 peut être directement formée dans le couvercle 12.

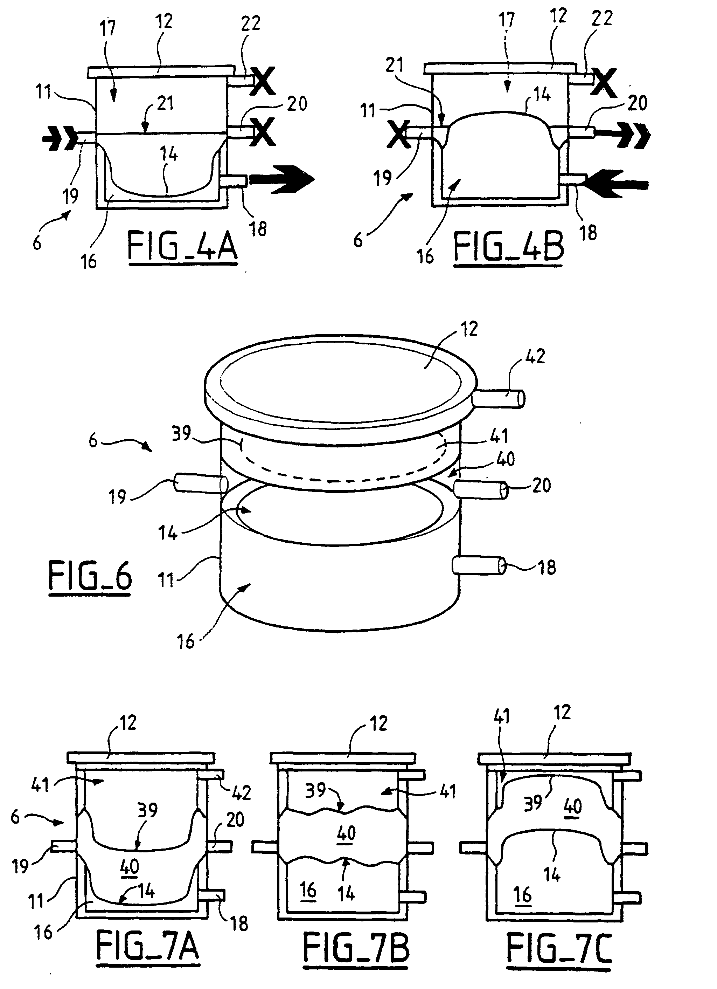

Un exemple d'alimentation de la chambre 6 en fluide de culture est illustré sur la figure 4A. Le fluide de culture est avantageusement introduit par l'entrée 19 dans la partie supérieure 17 du réceptacle 11, alors qu'une dépression est formée à l'intérieur de la partie inférieure 16 de ce même réceptacle 11, par extraction du gaz via l'entrée/sortie 18.

Un exemple d'évacuation du fluide de culture usé est illustré sur la figure 4B. Dans ce mode d'évacuation, on ouvre la vanne qui contrôle l'accès à la sortie 20, tout en introduisant un gaz haute pression à l'intérieur de la partie inférieure 16.

Comme cela est mieux illustré sur la figure 1, la chambre de culture peut comporter une troisième entrée 23 raccordée à une branche auxiliaire 24 du circuit de pressurisation 2. Cette branche auxiliaire 24 débouche dans un réservoir de gaz 25 logé dans le premier compartiment 1 du dispositif. Ce réservoir de gaz 25 est alimenté en gaz choisi par une, ou deux lignes 26 et 27 d'alimentation en gaz dont les accès sont respectivement contrôlés par des électrovannes 28 et 29 pilotées par les moyens de commande 3 et 4. Chaque ligne d'alimentation 26 et 27 est raccordée à un réservoir externe de gaz, contenant, par exemple, du gaz carbonique ou de l'oxygène, ou encore de l'air. De préférence, les gaz qui sont introduits dans le réservoir de gaz 25, via les lignes 26 et 27, et qui sont destinés à être introduits à l'intérieur de la partie inférieure 16, sont choisis de manière à favoriser la culture des cellules et tissus à l'intérieur de la chambre 6. En d'autres termes, ces gaz ont une double fonction : assurer la pressurisation à l'intérieur de la partie inférieure 16 et participer à la culture des cellules et des tissus à l'intérieur de la partie supérieure 17, au même titre que le fluide de culture. Pour ce faire, la membrane déformable 14 est avantageusement poreuse, au moins dans le sens allant de la partie inférieure 16 vers la partie supérieure 17. De plus, cette porosité est choisie conjointement avec la haute pression du gaz, de sorte que le débit d'entrée du gaz à l'intérieur de la partie supérieure 17 soit bien contrôlé.

Bien entendu, dans une variante, la membrane 14 pourrait ne pas être poreuse. Dans ce cas, la chambre 6 devrait comporter, dans sa partie supérieure 17 une entrée supplémentaire destinée à l'introduction de gaz de culture. La branche principale 99 du circuit de pressurisation 2 est raccordée, d'une part, à un compresseur 30 et, d'autre part, au réservoir de gaz 25. Ce compresseur 30 est alimenté en gaz, basse pression, par le réservoir de gaz 25. De manière générale, sur les figures, les parties du circuit de pressurisation qui se trouvent alimentées en gaz basse pression sont matérialisées par des tirets, tandis que les parties de ce circuit alimentées en gaz haute pression sont matérialisées par des traits continus. Le compresseur 30 est bien entendu piloté par les moyens de commande 4.

Grâce aux branches principale 99 et secondaire 24 du circuit de pressurisation 2, ainsi qu'au compresseur 30, qui alimente ladite branche principale 99, il est possible de fournir à la partie inférieure 16 de la chambre 6 différentes pressions de gaz, selon les besoins.

Le dispositif peut également comporter une ligne d'évacuation 31 destinée à évacuer tout ou partie du gaz contenu à l'intérieur du réservoir de gaz 25. Cette ligne d'évacuation 31 est avantageusement à accès contrôlé par une vanne 32 pilotée par les moyens de commande 4. Il est ainsi possible de purger, lorsque le besoin s'en fait sentir, tout ou partie du contenu du réservoir de gaz 25.

Par ailleurs, il est particulièrement avantageux que le dispositif selon l'invention comprenne un moyen de chauffage 33 destiné à réchauffer le gaz contenu à l'intérieur du réservoir de gaz 25. Cela permet en effet de réguler la température à l'intérieur de la chambre 6, par échange thermique entre le gaz et le fluide de culture. Ce moyen de chauffage 33 est, par exemple, réalisé sous la forme d'une résistance chauffante logée à l'intérieur du réservoir de gaz 25 et contrôlé par les moyens de commande 3. La régulation de la température s'effectue, dans ce cas, grâce à des mesures de température délivrées aux moyens de commande 3 et 4 par un capteur de température 34 également logé à l'intérieur du réservoir 25, de préférence. Mais, ce capteur peut être logé dans la chambre 6.

On peut également prévoir, à l'intérieur du réservoir de gaz 25, un analyseur de gaz 35, destiné à délivrer aux moyens de commande 3 des mesures représentatives de la proportion (ou pourcentage) de certaines molécules à l'intérieur du réservoir de gaz 25. Cet analyseur peut également servir à déterminer la consommation de gaz dans la chambre. De la sorte, les moyens de commande 3 et 4 peuvent gérer, avec précision, la composition du gaz qui est introduit dans la partie inférieure 16 de la chambre 6, et qui, lorsque la membrane est poreuse, est destiné à être mélangé au fluide de culture à l'intérieur de la partie supérieure 17.

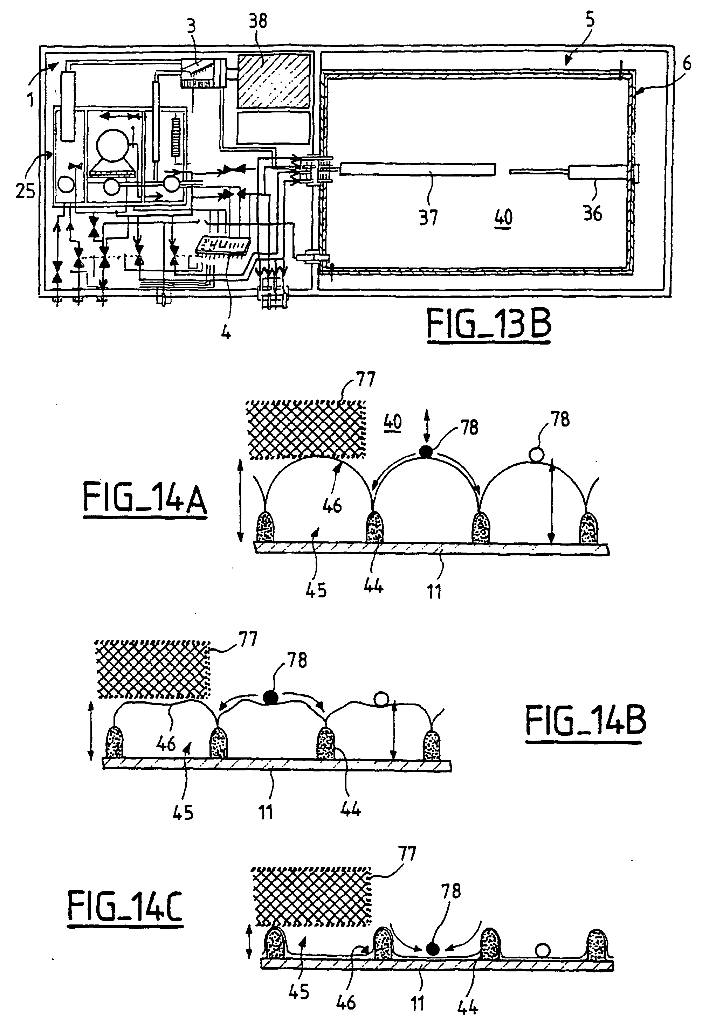

Comme on peut le voir sur la figure 13B, on pourra également prévoir à l'intérieur de la partie supérieure 17 de la chambre 6 un autre capteur de température 36 et/ou un capteur de pH. D'autres capteurs peuvent être également envisagés.

En résumé, le dispositif peut fonctionner, soit dans un mode préprogrammé simple, soit dans un mode préprogrammé avec autorégulation, selon qu'il comporte ou non des capteurs lui permettant de gérer la température des parties inférieure 16 et supérieure 17 de la chambre 6 et/ou les compositions gazeuses de ces deux parties.

Dans le mode préprogrammé, les moyens de commande 4 sont, avant le début d'une culture, alimentés en données destinées à assurer le contrôle de la culture. Ces données sont stockées dans une mémoire réinscriptible. En variante, lorsque le dispositif n'est destiné qu'à effectuer un seul et unique type de culture, les moyens de commande peuvent ne comporter qu'une mémoire non réinscriptible, ou bien directement des circuits hardware. Dans le cas d'une programmation, comme illustré sur la figure 1, le dispositif comporte avantageusement une interface 38, par exemple de type RS232 raccordée aux moyens de commande 3 et 4. Une telle programmation peut s'effectuer, par exemple, par l'introduction d'une suite de multiplets qui définissent la succession d'opérations qui doivent être effectuées par les moyens de commande pour assurer, dans des conditions adéquates, la culture des cellules et/ou des tissus à l'intérieur de la chambre de culture 6.

Chaque multiplet pourra comporter, par exemple, au moins un type de gaz choisi, une pression pour ce gaz choisi, une durée d'alimentation en ce gaz choisi, un débit de fluide de culture et une durée d'alimentation en ce fluide de culture. L'enchaînement d'opérations associées à chacun de ces multiplets, tel que défini par la suite programmée, assure la culture des cellules et/ou tissus.

Dans le mode de fonctionnement préprogrammé à autorégulation, les moyens de commande sont capables, sur la base des mesures qui leur sont délivrées par les différents capteurs, de modifier les paramètres du multiplet en cours de traitement, de manière à optimiser les conditions de culture à l'intérieur de la chambre. Cela peut consister, par exemple, en une modification du gaz introduit dans la première partie inférieure 16, ou bien une variation de la température de ce gaz, ou les deux en même temps, ou bien encore en une augmentation ou une réduction des durées d'introduction respectives du gaz et du fluide de culture.

Les moyens de commande pourront être constitués d'un microprocesseur 4 à mémoire RAM et/ou ROM, selon les besoins, couplé à une carte numérique 3 assurant le traitement des signaux issus des capteurs et analyseurs.

On se réfère maintenant aux figures 5A et 5B pour décrire une variante de chambre de culture pour un dispositif selon l'invention. Ce qui différencie cette chambre de culture de celle illustrée sur la figure 2, c'est le fait qu'elle contient au-dessus de la membrane inférieure 14, c'est à dire du côté opposé à la seconde partie inférieure 16, une sur-membrane déformable 90 délimitant avec cette membrane inférieure 14 une zone intermédiaire 91. En fait, dans l'exemple illustré, la sur-membrane 90 est solidarisée à la membrane inférieure 14 en une multiplicité d'endroits, de manière à subdiviser la zone intermédiaire 91 en une pluralité d'alvéoles 92 communicantes à volumes variables. La chambre 6 comprend, au niveau de la partie supérieure 17, au moins une entrée 93 raccordée à une sortie du circuit de pressurisation 2 dont l'accès est contrôlé par une vanne pilotée par le module de commande 3, 4, pour alimenter en gaz la zone intermédiaire 91 selon une pression choisie, un débit choisi et des durées choisies, en fonction des critères choisis, ainsi qu'au moins une sortie 94 pour évacuer de cette zone intermédiaire 91 une partie au moins du gaz. Cette sortie 94 est également à accès contrôlé par une vanne pilotée par le module de commande 3,4, et communique de préférence avec le circuit de pressurisation 2. Cela permet de gérer conjointement les formes des membrane 14 et sur-membrane 90 et la circulation du fluide de culture dans la première partie 17.

Le gaz qui alimente la zone intermédiaire peut être le gaz choisi qui alimente la partie inférieure 16, ou bien un autre gaz. Dans ce dernier cas, le circuit de pressurisation doit être adapté de manière à permettre une double alimentation en parallèle avec deux gaz différents. En soumettant la sur-membrane 90 à des cycles de pressions alternativement haute et basse, on peut instaurer un mode de battements (ou pulsations) particulièrement utile lorsqu'il faut reproduire une culture de tissus du type des tissus cardiaques.

Dans cette variante, la déformation de la membrane 14 assure des macro-mouvements, alors que la déformation de la sur-membrane 90 assure des micro-mouvements.

On se réfère maintenant aux figures 6 à 8 pour décrire une autre variante de chambre de culture pour un dispositif selon l'invention. Ce qui différencie cette chambre de culture de celle illustrée sur la figure 2, c'est le fait qu'elle contient dans la partie supérieure 17 une autre membrane 39 (supérieure) destinée à gérer, conjointement avec la membrane 14 (inférieure), la circulation du fluide à l'intérieur de la chambre 6. Cette membrane supérieure 39 est, comme la membrane inférieure 14, maintenue par ses bords à un niveau choisi par rapport au couvercle 12. Ainsi, un gaz peut être introduit, avec un débit, une durée d'alimentation et une pression choisis entre cette membrane supérieure 39 et le couvercle 12 de manière à gérer la déformation de la membrane supérieure. La membrane supérieure 39 forme une interface dans la partie supérieure 17 de la chambre 6, entre une partie centrale 40, dans laquelle se trouvent placés les cellules et/ou les tissus et dans laquelle est injecté le fluide de culture, et une partie arrière 41 dans laquelle est injecté le gaz gérant la forme de la membrane 39, par une entrée/sortie 42, de préférence formée dans le couvercle 12. Cette membrane supérieure 39 peut être supportée par des moyens de support solidarisés au réceptacle 11, dans sa partie supérieure, ou bien directement solidarisés au couvercle 12. De préférence, le gaz qui est utilisé pour déformer la membrane supérieure 39 est identique à celui utilisé pour déformer la membrane inférieure 14. Mais, il pourrait être également différent, moyennant les adaptations nécessaires de la chambre de culture et du circuit de pressurisation (deux branches d'alimentation parallèles couplées chacune à des vannes d'entrée et de sortie). De même, cette membrane supérieure 39 est de préférence poreuse, au moins dans le sens allant de la partie arrière 41 vers la partie centrale 40, de sorte que le gaz introduit dans cette partie arrière 41 puisse alimenter la partie centrale 40 en vue de la culture des tissus et/ou cellules.

De manière générale, la porosité des membranes (ou poches) est choisie de sorte qu'elle ne concerne que les gaz et non les cellules. En conséquence, il peut être avantageux que les membranes utilisée dans le dispositif soient poreuses dans les deux sens, permettant ainsi au) formés dans la partie supérieure du fait de la culture d'être évacués dans la partie inférieure 16, notamment.

Dans l'exemple illustré sur les figures 7A à 7C, les deux membranes supérieure 39 et inférieure 14 sont soumises à des pressions qui sont sensiblement en opposition de phase. En d'autres termes, lorsque l'une des membranes est en dépression, l'autre est en surpression. Mais il pourrait en être autrement, selon les besoins de la culture. Dans ce cas, la circulation du fluide dans la chambre 6 est essentiellement verticale.

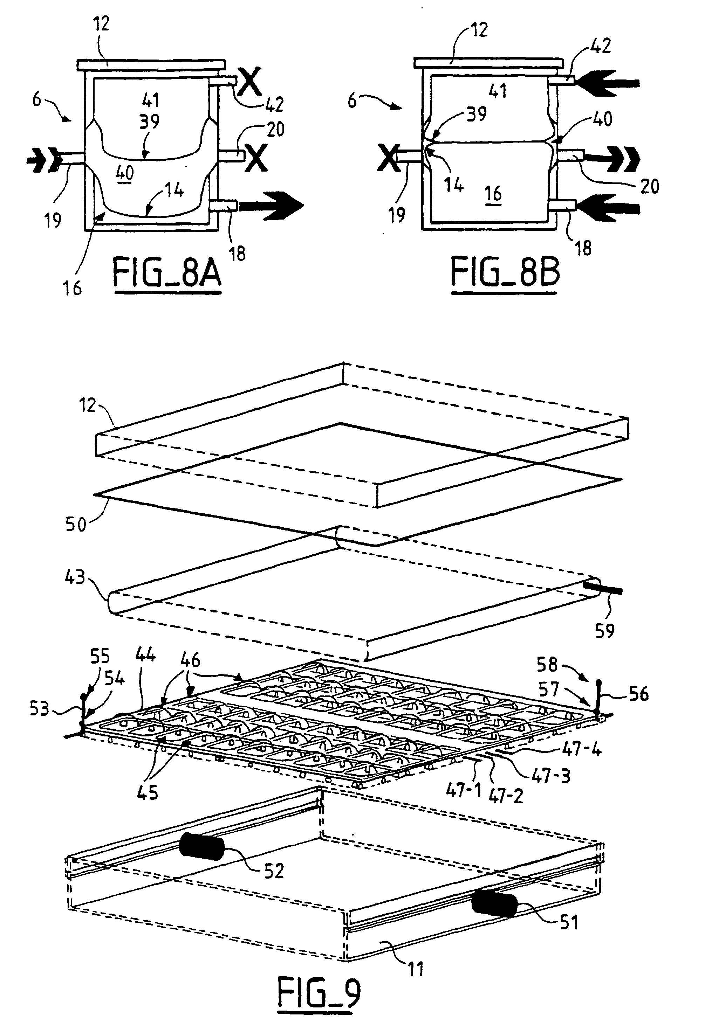

Sur la figure 8A se trouve illustré un exemple d'alimentation de la chambre 6 en fluide de culture. Dans cet exemple, le fluide de culture est introduit par l'entrée 19 à l'intérieur de la partie centrale 40, tandis que le gaz est extrait de la première partie inférieure 16 par ouverture de la vanne qui en contrôle l'accès. Dans le même temps, la vanne qui contrôle l'accès à l'entrée/sortie 42 de la partie arrière 41 est fermée.

Sur la figure 8B se trouve illustré un exemple d'évacuation de fluide de culture usé. Dans cet exemple, les deux membranes supérieure 39 et inférieure 14 sont soumises simultanément à des hautes pressions, par l'ouverture des vannes contrôlant les accès aux entrées 18 et 42, tandis que la vanne contrôlant l'accès à la sortie 20 est ouverte. Dans cette variante, il est possible de prévoir une sur-membrane 91 sur la membrane inférieure 14 (comme dans l'exemple illustré sur la figure 5), et/ou sur la membrane supérieure 39 (moyennant bien entendu les aménagements nécessaires de la chambre et du circuit de pressurisation).

On se réfère maintenant à la figure 9 pour décrire une autre variante de chambre de culture pour un dispositif de culture selon l'invention. Dans ce mode de réalisation, la chambre de culture 6 est également délimitée par un réceptacle 11 coopérant avec un couvercle 12. La membrane supérieure 39 de la chambre de la figure 6, est ici remplacée par une poche déformable 43 qui peut être solidarisée, soit à la partie supérieure 17 du réceptacle 11, soit directement au couvercle 12. Par ailleurs, au lieu de loger une unique membrane inférieure déformable 14, le réceptacle 11 loge une multiplicité de membranes inférieures déformables indépendamment les unes des autres. Pour ce faire, les moyens de support des membranes inférieures sont réalisés sous la forme d'un bâti 44 dans lequel se trouve définie une multiplicité de cavités 45. Chaque cavité 45 est propre à supporter à étanchéité le bord périphérique d'une membrane inférieure 46, à être alimentée en gaz, du côté de la partie inférieure 16 et à loger une membrane lorsque la pression du gaz à l'intérieur de la partie inférieure 16 est inférieure à un seuil, par exemple lorsque le gaz est en basse pression (dépression).

Un tel bâti est, par exemple, réalisé dans un matériau élastomère dans lequel se trouvent intégrés des conduits d'alimentation en gaz (non représentés) raccordés au circuit de pressurisation 2 et alimentant les cavités soit individuellement, soit collectivement (dans ce cas, il est avantageux que les cavités d'un même groupe communiquent entre elles).

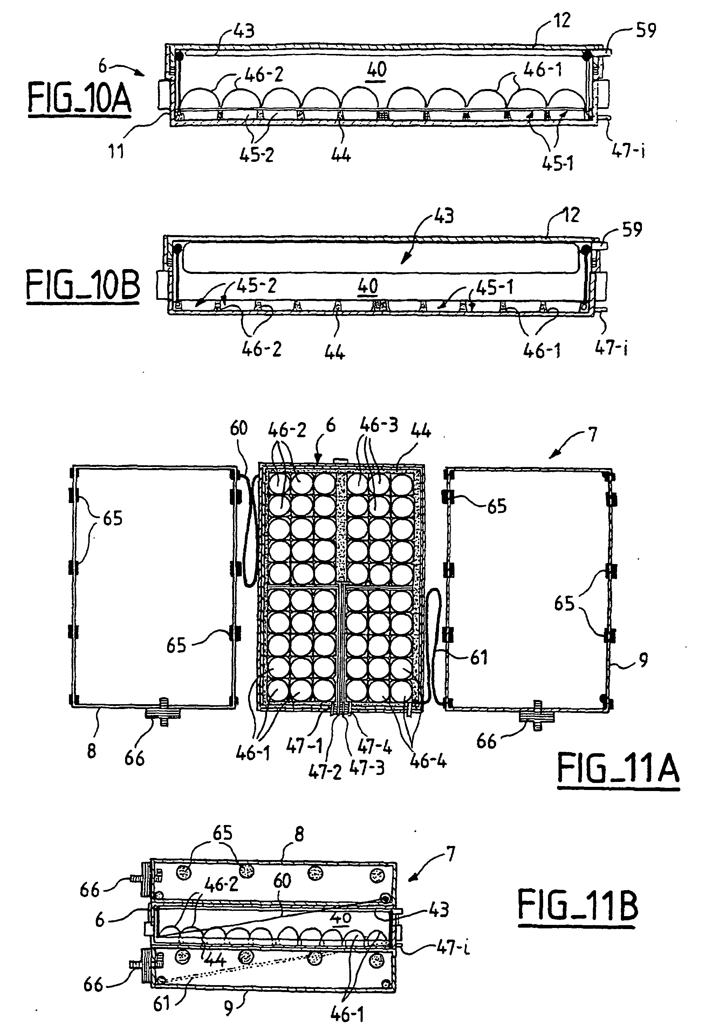

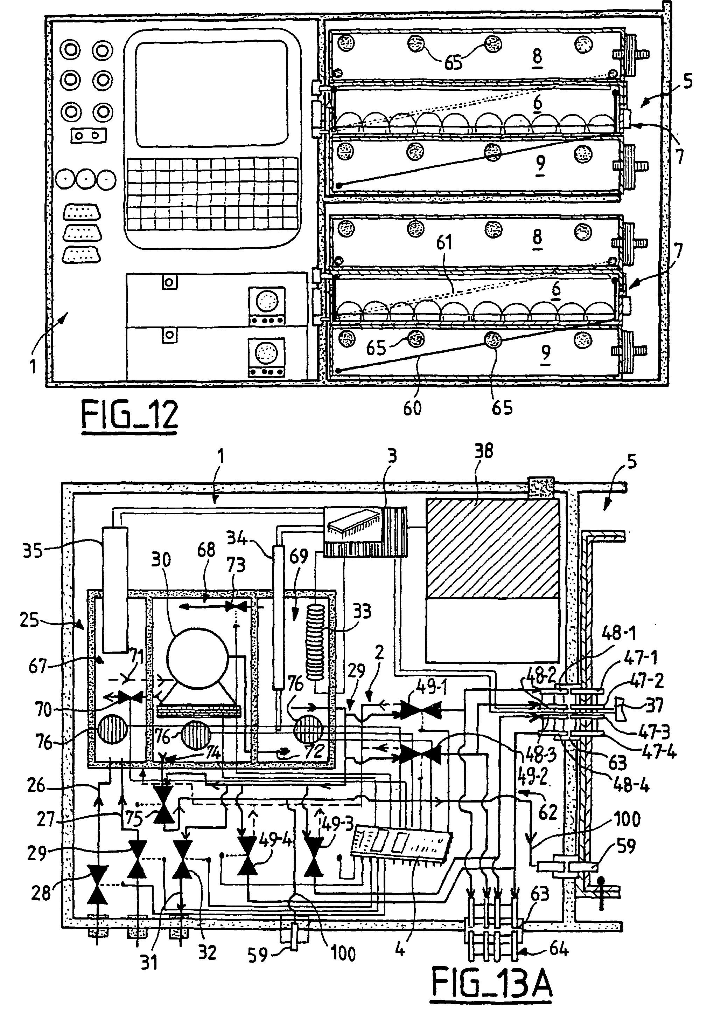

Cette multiplicité de membranes inférieures 46 est avantageusement subdivisée en deux groupes, ou plus, par exemple trois, voire quatre, comme illustré sur la figure 11A. Ainsi, toutes les membranes inférieures appartenant à un même groupe peuvent être soumises à des conditions de pression sensiblement identiques, ces conditions pouvant être différentes d'un groupe à l'autre. Dans l'exemple, le bâti comporte quatre conduits principaux 47-1 à 47-4 (voir figure 11A) destinés à alimenter respectivement les quatre groupes de membranes inférieures 46-1 à 46-4. Dans ce cas, et comme cela est mieux illustré sur la figure 13, le circuit de pressurisation 2 comporte quatre sorties d'alimentation en gaz 48-1 à 48-4 (formant sortie de la branche principale 99), chacune de ces sorties 48-i (i = 1 à 4, pour cet exemple) étant à accès contrôlé par une électrovanne 49-i pilotée par les moyens de commande 3 et 4. Ainsi, chaque groupe de membranes inférieures 46-i peut être géré indépendamment des autres groupes, par une préprogrammation appropriée. En d'autres termes, la programmation des opérations nécessaires à une culture donnée consiste en la mémorisation dans les moyens de commande d'une suite de multiplets du type de ceux décrits précédemment, mais comportant chacun des paramètres propres à chaque groupe.

Comme illustré sur la figure 9, le réceptacle 11 de la chambre de culture 6 comporte, de préférence, une ouverture 51 adaptée à l'introduction, à étanchéité, du capteur de température 36, ainsi qu'une seconde ouverture 52 adaptée à l'introduction à étanchéité du capteur de pH 37, à l'intérieur de la partie centrale 40 définie dans la partie supérieure 17 par la poche 43. Bien entendu, ce réceptacle 11 comporte également des ouvertures permettant le passage des extrémités des conduits 47-i d'alimentation et d'extraction en gaz.
D'autre part, dans le mode de réalisation illustré sur la figure 9, notamment, l'alimentation en fluide de culture s'effectue, de préférence, à deux niveaux différents. Pour ce faire, le bâti 44 est avantageusement équipé d'un conduit 53 comportant une partie sensiblement verticale munie de deux sorties 54 et 55 destinées respectivement à délivrer le fluide de culture au niveau des membranes inférieures et de la poche déformable 43. Cela permet une meilleure répartition du fluide de culture à l'intérieur de la partie centrale 40. De même, on prévoit avantageusement un conduit 56, à deux entrées 57 et 58 placées à des niveaux différents, de manière à permettre l'évacuation du fluide de culture usé, respectivement au niveau des membranes inférieures 46-i et de la poche déformable 43. Ces deux entrées 57 et 58 peuvent alimenter deux conduits d'évacuation, au lieu d'un seul, chaque conduit étant à accès contrôlé par une vanne pilotée par les moyens de commande ou bien actionnable manuellement. Cela permet, dans le mode monophase d'évacuer les gaz résiduels qui se concentrent au-dessus du fluide de culture, et dans le mode biphase d'extraire sélectivement du gaz et/ou du fluide de culture.

La poche supérieure déformable 43 est, de préférence, réalisée sous la forme d'une membrane poreuse, au moins dans le sens allant de l'intérieur de la poche (ou partie arrière 41) vers la partie centrale 40. Elle peut être réalisée dans le même matériau que la ou les membranes inférieure(s) et supérieure(s). Cette poche agit, en fait, comme la membrane supérieure 39 décrite précédemment en référence à la figure 6, notamment. Elle comporte de ce fait une entrée/sortie 59 équivalente à l'entrée/sortie 42 et destinée à permettre l'introduction du gaz et son extraction sur ordre des moyens de commande. Ainsi, en contrôlant les alimentations en gaz de la poche supérieure déformable 43 et/ou des membranes inférieures 46-i, il est possible de contrôler la circulation du fluide à l'intérieur de la partie centrale 40 de la chambre de culture 6.

Préférentiellement, comme illustré sur les figures 11A et 11B, la chambre de culture 6 fait partie d'une unité de culture autonome 7, conjointement avec les réservoirs d'alimentation 8 et de collection 9. Dans ce cas, la chambre 6 est raccordée à chaque réservoir par l'intermédiaire d'un conduit 60, 61 dont la longueur est adaptée de manière à permettre l'empilement des deux réservoirs de part et d'autre de la chambre de culture 6, comme illustré sur la figure 11B. Cette solution est particulièrement intéressante, dans la mesure où elle assure une compacité de l'unité de culture 7.

Comme cela est illustré sur les figures 1 et 12, chaque unité de culture 7 peut être logée à l'intérieur d'un compartiment 5 du dispositif selon l'invention. Un tel compartiment 5 peut éventuellement (comme illustré sur la figure 12) loger deux unités de culture 7, voire plus.

Par ailleurs, et comme cela est mieux illustré sur la figure 13A, le dispositif selon l'invention, et notamment son compartiment 1, peut être adapté pour être raccordé à une chambre de culture externe, ou bien à une unité de culture externe, voire à plusieurs. Pour ce faire, le circuit de pressurisation 2 comporte une partie de dérivation 62 comprenant quatre branches de dérivation raccordées respectivement aux électrovannes 49-1 à 49-4, et par conséquent pouvant être alimentée en gaz, de la même façon que les sorties 48-1 à 48-4 de la branche principale 99 du circuit de pressurisation 2. Mais, ces branches de dérivation pourraient posséder leurs propres électrovannes de contrôle d'accès. Préférentiellement, le raccordement d'une unité de culture 7 au circuit de pressurisation 2 s'effectue par l'intermédiaire d'une interface 63. Ainsi, il est particulièrement aisé de connecter une nouvelle unité en vue d'une nouvelle culture. D'autre part, au lieu de loger une unité de culture complète à l'intérieur du second compartiment 5 du dispositif, on pourrait ne loger qu'une ou plusieurs chambres de culture 6, celles-ci étant alors raccordées à des réservoirs externes. Enfin, les réservoirs 8 et 9 comportent, de préférence, plusieurs ouvertures étanches adaptées pour permettre l'introduction (ou l'extraction) du fluide de culture, ou de tout autre produit. De telles ouvertures sont par exemple équipées de septum 65. Par ailleurs, afin de maintenir une pression atmosphérique à l'intérieur des réservoirs, ceux-ci peuvent être équipés d'ouvertures munies de filtres 66.

D'autre part, et bien que cela ne soit pas représenté sur les figures, les réservoirs peuvent être munis de plusieurs poches souples contenant des fluides de culture différents, de préférence sensiblement à la pression atmosphérique. Dans ce cas, les poches sont soit raccordées à un distributeur piloté par les moyens de commande ou manuellement, soit raccordées chacune à un conduit d'alimentation qui débouche dans la partie supérieure 17 de la chambre 6 et dont l'accès est contrôlé par les moyens de commande ou manuellement.

On se réfère maintenant plus particulièrement à la figure 13A pour décrire une variante de réalisation du circuit de pressurisation du dispositif selon l'invention. Dans cette variante, le réservoir de gaz 25 est subdivisé en trois sous-parties 67, 68 et 69, dans lesquelles règnent respectivement une basse pression (sensiblement la pression atmosphérique), une pression intermédiaire et une haute pression. La première sous-partie 67 est raccordée aux conduits 26 et 27 d'alimentation en gaz. Elle communique par une ouverture à accès contrôlé par une vanne 70, avec la seconde sous-partie 68. La seconde sous-partie 68 loge, de préférence, le compresseur 30, lequel est alimenté par un conduit 71 qui débouche dans la première sous-partie 67. Le compresseur 30 alimente par un conduit 72 la troisième sous-partie 69. Cette seconde sous-partie communique également avec la troisième sous-partie 69 par une ouverture à accès contrôlé par une vanne 73. Les deux vannes 70 et 73 permettent ainsi d'instaurer des pressions choisies à l'intérieur de chacune des trois sous-parties 67 à 69, sous contrôle des moyens de commande 3 et 4. La troisième sous-partie 69 est raccordée au circuit de pressurisation, et notamment à sa branche principale 99 pour l'alimentation en gaz haute pression. La partie du circuit de pressurisation 2 qui est utilisée pour évacuer le gaz qui se trouve à l'intérieur de la partie inférieure 16 de chaque chambre de culture 6 débouche dans la première sous-partie 67, si bien que le circuit de pressurisation 2 est un circuit de type fermé.

On peut prévoir au niveau de la seconde sous-partie 68, placée à une pression intermédiaire, une sortie 74 alimentant une vanne 75 dont la sortie 100 est raccordée à l'entrée 59 de la poche supérieure 43 (ou bien à la partie arrière 41 lorsque la poche est remplacée par une membrane supérieure 39). Cette vanne 75 est, de préférence, également alimentée en gaz basse pression et haute pression issus des première 67 et troisième 69 sous-parties du réservoir de gaz 25, de sorte que la poche 59 (ou l'arrière de la membrane supérieure 39) puisse être directement alimentée en un gaz à la pression choisie, sans passer par le compresseur 30.

Lorsque le dispositif est adapté à l'autorégulation, il est particulièrement avantageux que l'analyseur de molécules 35 soit logé à l'intérieur de la première sous-partie 67, et que la résistance de chauffage 33 et le capteur de température 34 soient logés dans la troisième sous-partie 69. D'autre part, il est également avantageux que chacune des trois sous-parties 67 à 69 soit équipée d'un capteur de pression 76 destiné à délivrer ses mesures de pression internes aux moyens de commande 3 et 4, de sorte que ceux-ci puissent contrôler avec précision les pressions régnant dans chacune des sous-parties.

On se réfère maintenant plus particulièrement aux figures 14 à 21 pour décrire différents modes de circulation du fluide de culture à l'intérieur de la chambre de culture 6.

Comme exposé précédemment, le dispositif selon l'invention est destiné à faire croître dans la partie centrale 40 de la chambre de culture 6 des tissus et/ou des cellules 78 grâce à un environnement contrôlé, composé de fluide de culture mélangé à un ou plusieurs gaz choisis. Pour permettre une croissance optimale de ces tissus et/ou cellules, il est nécessaire que le fluide de culture se déplace relativement aux cellules et/ou tissus. Comme illustré sur les figures 14A à 14C, un simple déplacement alternatif, vertical, des membranes déformables inférieures 46 permet de déplacer verticalement, et latéralement, les cellules à l'intérieur de la partie centrale 40 de la chambre de culture 6. La multiplicité de membranes inférieures 46 permet, notamment lorsque les membranes se trouvent dans leurs deux états de déformation extrême (figures 14A et 14C), aux cellules et tissus de se rapprocher les uns des autres, soit parce qu'ils se trouvent dans une position d'équilibre instable, soit parce qu'ils sont contraints à se rassembler à l'intérieur des cavités 45 définies par le bâti 44. Les cellules 78 peuvent être ainsi déplacées, quelle que soit leur position dans le bain de culture, empêchant ainsi leur accumulation en une région d'étendue limitée. Un mouvement rapide de gonflement et de dégonflement des membranes inférieures augmente considérablement l'homogénéité de la répartition des cellules dans le milieu de culture (fluide de culture plus gaz). En revanche, un mouvement lent de gonflement et dégonflement peut permettre la création de zones de concentrations cellulaires différentes à l'intérieur de la chambre.

Le dispositif selon l'invention convient tout particulièrement à la culture de cellules en trois dimensions sur un support 77, et notamment la culture de tissus. De tels supports 77 peuvent, quelle que soit leur nature, être placés directement sur les membranes inférieures 46-i, si bien que le déplacement de ces membranes inférieures crée une agitation efficace, susceptible de réduire de façon significative les forces de cisaillement. De la sorte, il est offert un environnement particulièrement adapté à la culture des cellules à forte densité cellulaire.

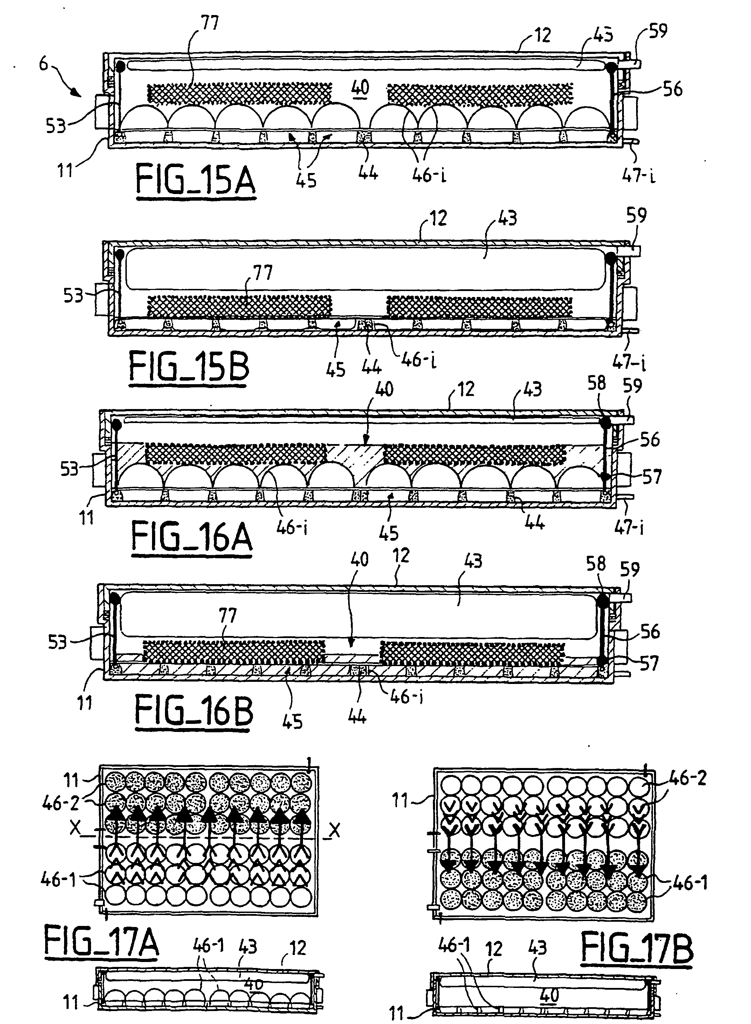

Comme illustré sur les figures 15 et 16, plusieurs supports de culture 77 peuvent être logés à l'intérieur d'une chambre de culture. Notamment, lorsque la multiplicité de membranes inférieures est subdivisée en groupes, il est possible de prévoir un support 77 pour chaque groupe. De tels supports de culture 77 peuvent présenter des géométries et des natures différentes, selon les besoins. Il pourra s'agir, par exemple, de sphéroïdes (pré-tissus en forme de sphère) ou de supports micro-porteurs en suspension. Il peut être également envisagé de placer les supports 77, individuellement, à l'intérieur d'une enveloppe poreuse placée dans la partie centrale 40, cette enveloppe étant poreuse vis-à-vis du milieu de culture, mais pas vis-à-vis des cellules. Ainsi, les cellules directement injectées à l'intérieur de l'enveloppe peuvent continuer à vivre et proliférer tout en restant confinées sur le support. Un tel mode de réalisation permet de mettre en culture à l'intérieur d'une même chambre 6, plusieurs types de cellules ou de tissus, sans risque de contamination croisée. Bien entendu, il est également possible de cultiver simultanément à l'intérieur d'une même chambre de culture 6, des cellules et/ou des tissus confinés à l'intérieur d'enveloppes, et des cellules et/ou tissus non confinés. Une telle diversité de cultures permet de simuler des environnements de type in vivo.

Comme cela est illustré sur les figures 15 et 16, le dispositif selon l'invention peut assurer une culture de type monophase (figures 15A et 15B) ou biphase (figures 16A et 16B). Dans le cas d'une culture monophase, la quasi-totalité de la partie centrale 40 de la chambre de culture 6 contient un fluide de culture, en phase liquide, si bien que le support de culture 77 et les tissus et/ou cellules à cultiver, sont de façon, quasi permanente, immergés dans un liquide de culture. Mais, pour certains types de culture, il est primordial que les tissus et/ou cellules ne soient pas continuellement immergés dans le liquide de culture. Dans ce cas, un fonctionnement en mode biphase, du type de celui illustré sur les figures 16A et 16B, est particulièrement approprié. Ce mode consiste à introduire une quantité choisie, limitée, de gaz, comme par exemple de l'air, au-dessus de la phase liquide (matérialisée par des parties grisées). Le déplacement des membranes inférieures, couplé, éventuellement, aux déformations de la poche supérieure 43, permet d'exposer les cellules qui se sont fixées sur un support de culture 77 alternativement à la phase liquide et à la phase gazeuse. En faisant varier la quantité d'air et la fréquence des mouvements des membranes inférieures 46 et/ou de la poche supérieure 43, le dispositif peut contrôler la durée de l'exposition des cellules et/ou tissus à la phase gazeuse.

Dans ce mode de fonctionnement, il est particulièrement intéressant que le conduit 56 permettant d'extraire le milieu de culture hors de la chambre de culture 6, comporte deux entrées 57 et 58 placées à des niveaux différents, l'une 57 placée sensiblement au niveau des membranes inférieures 46, et l'autre 58 placée sensiblement à proximité de la poche 43. Ainsi, l'entrée 57 du conduit d'évacuation 56 permet d'évacuer la phase liquide, tandis que l'entrée supérieure 58 de ce même conduit permet d'évacuer la phase gazeuse.

On se réfère maintenant plus particulièrement aux figures 17 à 21 pour décrire des exemples, non limitatifs, de mode de circulation du fluide de culture à l'intérieur de la partie centrale 40 de la chambre de culture 6.

Dans l'exemple illustré sur les figures 17A et 17B, la multiplicité de membranes inférieures est subdivisée en deux groupes 46-1 et 46-2 placés de part et d'autre d'un axe XX dans la chambre de culture 6. Le groupe de membranes inférieures placé à droite de cet axe XX est ici référencé 46-1 tandis que le groupe de membranes inférieures placé à gauche est référencé 46-2. En déplaçant en opposition de phase les membranes inférieures de droite 46-1 et les membranes inférieures de gauche 46-2, on contraint le milieu de culture à se déplacer alternativement de la gauche vers la droite puis de la droite vers la gauche. Cela assure une circulation essentiellement horizontale.

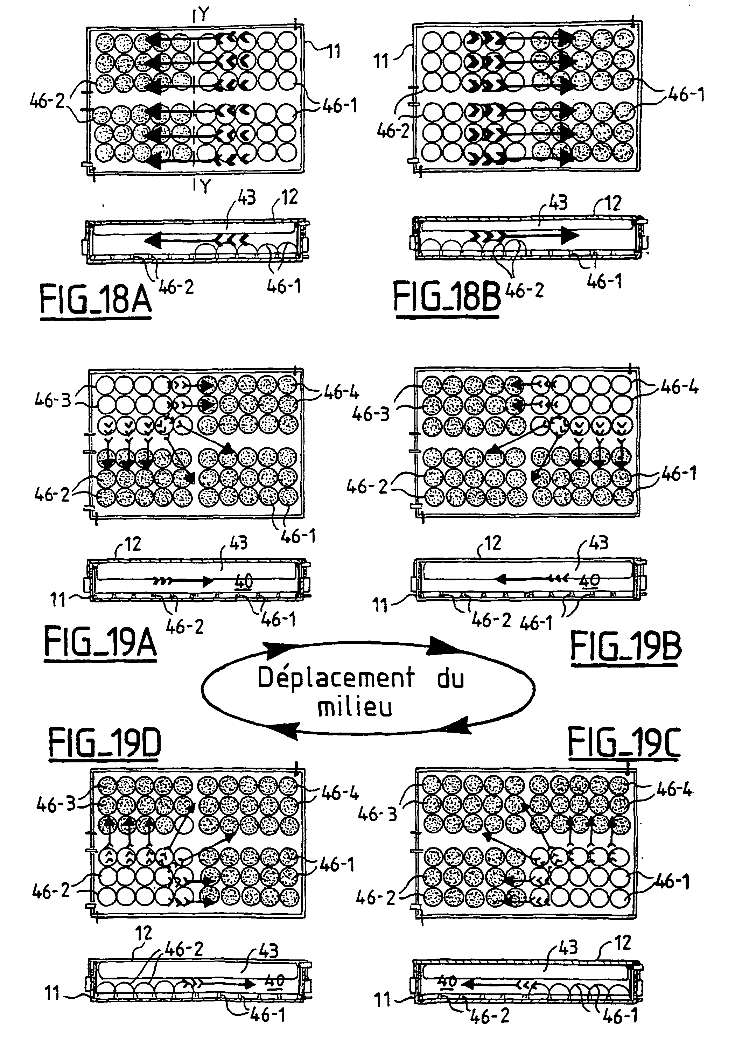

Dans l'exemple illustré sur les figures 18A et 18B, la multiplicité de membranes inférieures est également subdivisée en deux groupes 46-1 et 46-2 placés respectivement de part et d'autre d'un axe YY perpendiculaire à l'axe XX des figures 17. Les membranes inférieures 46-1 sont placées à droite de l'axe transversal YY, tandis que les membranes inférieures 46-2 sont placées à gauche de cet axe. En déplaçant en opposition de phase les membranes de droite 46-1 et les membranes de gauche 46-2, on contraint le fluide de culture à se déplacer alternativement de la droite vers la gauche puis de la gauche vers la droite, relativement à l'axe YY. Cela assure également une circulation essentiellement horizontale.

Dans les deux modes de circulation illustrés sur les figures 17 et 18, il n'est pas indispensable de faire varier l'état de gonflement de la poche déformable 43. Celle-ci peut être maintenue dans un état gonflé durant toute la suite d'alternance droite/gauche. Par ailleurs, dans ces deux cas, on peut subdiviser chaque groupe en sous-groupes de manière à gérer précisément le volume de culture dans la partie centrale 40. En effet, si par exemple on maintient dans l'état gonflé la poche 43 et l'un des groupes de membranes inférieures 46-i, on réduit le volume de culture dans la chambre, ce qui, du fait de la concentration du fluide de culture dans la partie dégonflée, conduit à une diminution de la concentration cellulaire dans ce fluide. Dans ce cas, les déplacements relatifs du fluide de culture et des cellules peuvent être assurés par la gestion des gonflements et dégonflements respectifs des sous-groupes du groupe qui n'est pas maintenu dans l'état gonflé.

Le mode de circulation illustré sur les figures 19A à 19D est un cycle à quatre étapes, nécessitant la subdivision de la multiplicité de membranes inférieures en quatre groupes 46-1 à 46-4. Pendant chaque étape, seul l'un des groupes de membranes est dans son état gonflé (membrane soumise à un gaz haute pression), tandis que les trois autres groupes sont dans un état dégonflé (membrane soumise à un gaz basse pression (ou dépression)). Ainsi, le fluide de culture, qui se trouve sensiblement placé dans la région située au-dessus du groupe de membranes inférieures qui passent de l'état dégonflé à l'état gonflé, est contraint à rejoindre les régions situées au-dessus des trois autres groupes dont les membranes passent de l'état gonflé à l'état dégonflé, ou demeurent dans l'état dégonflé. Cela assure un déplacement permanent du fluide de culture, dans le plan horizontal, dans toutes les directions.

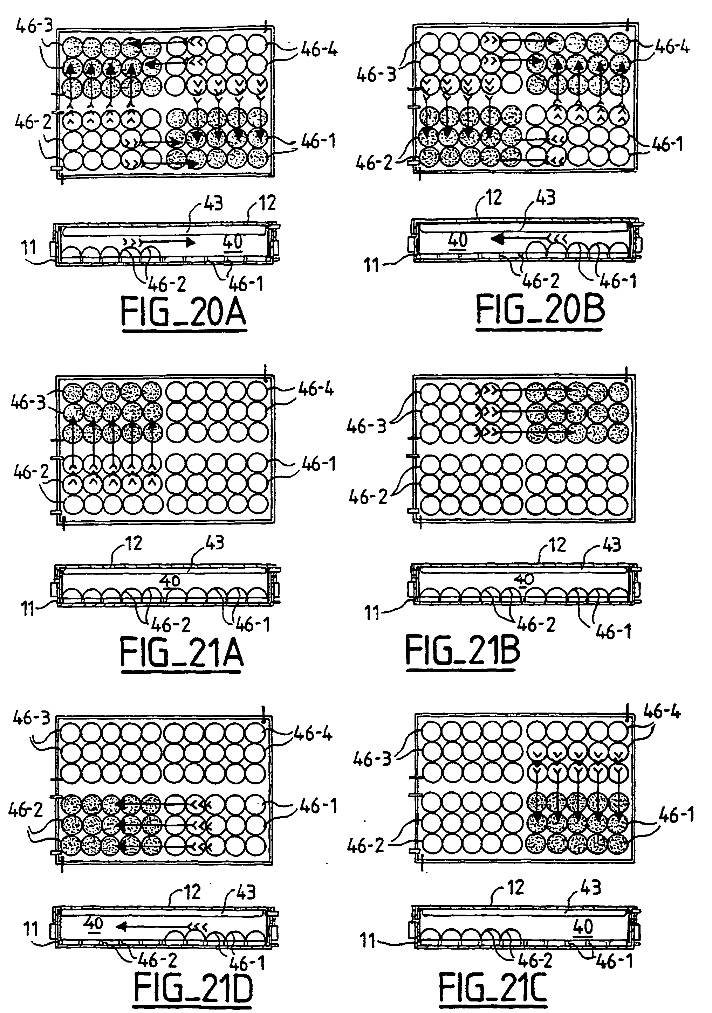

Le mode de circulation illustré sur les figures 20A et 20B est un cycle à deux étapes, dans lequel la multiplicité de membranes inférieures est également subdivisée en quatre groupes 46-i. Dans ce mode, les deux groupes de membranes inférieures 46-1 et 46-3 placés sensiblement sur une diagonale, sont déplacés en opposition de phase par rapport aux deux autres groupes 46-2 et 46-4 placés sensiblement suivant l'autre diagonale. Ainsi, l'état de chaque groupe de membranes inférieures alterne de l'état gonflé à l'état dégonflé, et le milieu de culture, qui se trouve dans la région placée sensiblement au-dessus des membranes inférieures dont l'état passe de l'état dégonflé à l'état gonflé, est contraint à rejoindre les deux régions qui se trouvent sensiblement placées au-dessus des membranes dont l'état varie de l'état gonflé à l'état dégonflé. Le milieu de culture est par conséquent contraint à se déplacer suivant deux directions sensiblement perpendiculaires entre elles, de la droite vers la gauche et d'avant en arrière. Dans les modes de circulation illustrés sur les figures 19 et 20, il est également préférable que la poche supérieure déformable 43 demeure dans son état gonflé pendant toute la durée des cycles ou bien dans un état «libre» dans lequel elle est sensiblement à la pression atmosphérique (étant alimentée directement par la première sous-partie 67 du réservoir de gaz 25). Ce dernier cas assure une autorégulation de la pression dans la chambre 6, du fait que le volume de la poche peut s'adapter automatiquement aux variations de pression dans ladite chambre.

Le mode de circulation illustré sur les figures 21A à 21D est un cycle à quatre étapes, assurant un déplacement circulaire, dans le plan horizontal du milieu de culture. Ce mode de circulation nécessite également une subdivision de la multiplicité de membranes inférieures en quatre groupes 46-1 à 46-4. Ici, à chaque étape, seul l'un des groupes de membranes est placé dans un état dégonflé, tandis que les trois autres groupes de membranes sont placés dans un état gonflé. Le fluide de culture, qui se trouve dans la région placée sensiblement au-dessus du groupe de membranes inférieures dont l'état passe de l'état dégonflé à l'état gonflé, est donc contraint à rejoindre la région qui se trouve sensiblement placée au-dessus des membranes inférieures dont l'état passe de l'état gonflé à l'état dégonflé. Par conséquent, le fluide de culture qui se déplace suivant une première direction, lors d'une première étape, est contraint à se déplacer suivant une seconde direction perpendiculaire à la première direction lors de la seconde étape et ainsi de suite. Ce mode convient tout particulièrement au déplacement de cellules , de sphéroïdes et micro-porteurs à l'intérieure de la partie centrale 40, sous réserve de l'observation de phases de décantation. Dans ce mode de circulation, il est préférable que la poche déformable supérieure 43 soit maintenue dans son état libre (défini ci-avant) pendant les cycles successifs.

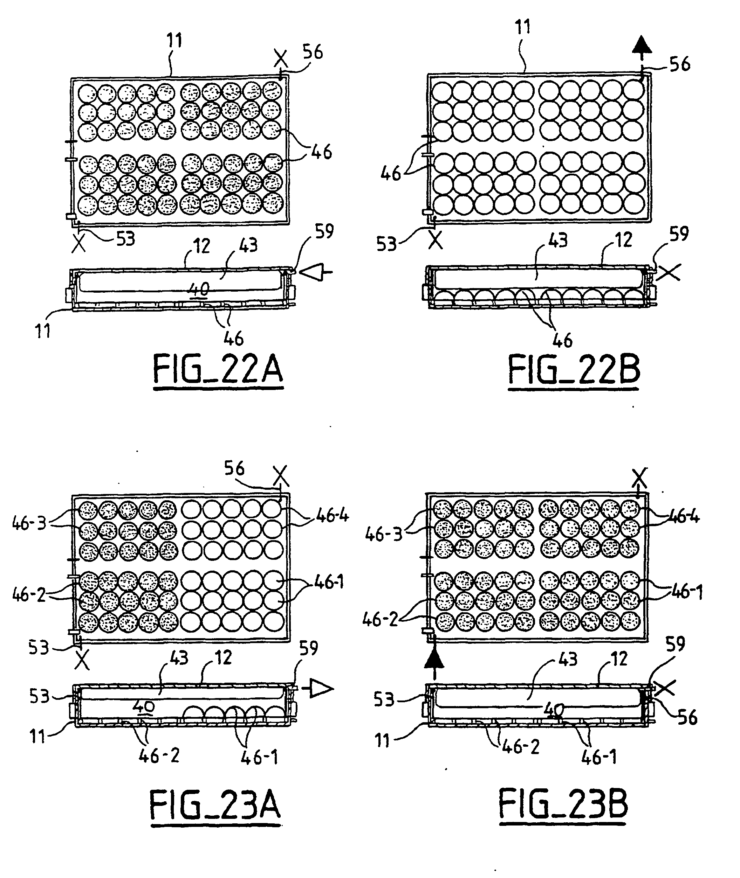

On se réfère maintenant aux figures 22A et 22B pour décrire un mode d'évacuation du fluide de culture usé.

Dans cet exemple, deux étapes successives peuvent être mises en oeuvre.

Dans la première étape (figure 22A), les vannes contrôlant l'entrée du conduit d'alimentation en fluide 53 et de la sortie du conduit d'évacuation de fluide 56 sont fermées. Toutes les membranes inférieures 46-i sont placées dans l'état dégonflé, tandis que la vanne contrôlant l'accès à la poche déformable supérieure 43 est ouverte. La poche 43 est donc contrainte à se gonfler au maximum. Dans la seconde étape (figure 22B), la vanne contrôlant l'entrée du conduit d'alimentation 53 est maintenue fermée, tandis que celle contrôlant la sortie du conduit d'évacuation 56 est ouverte. Dans le même temps, la vanne contrôlant l'accès à la poche déformable supérieure 43 est fermée, tandis que les membranes inférieures 46 sont placées dans leur état gonflé. Il en résulte que le fluide de culture est contraint à évacuer la chambre de culture 6 par le conduit 56.

On se réfère maintenant aux figures 23A et 23B pour décrire un mode d'alimentation de la chambre de culture en fluide de culture. Ce mode comporte également deux étapes. Dans une première étape (figure 23A), on maintient dans une position fermée les vannes d'accès à l'entrée du conduit d'alimentation 53 et à la sortie du conduit d'évacuation 56, tandis que la vanne d'accès à l'entrée/sortie 59 de la poche supérieure déformable 43 est placée dans une position ouverte. Dans le même temps, les deux groupes 46-2 et 46-3 de membranes inférieures, placés à gauche de l'axe transversal YY, sont placés dans l'état dégonflé, tandis que les deux groupes 46-1 et 46-4, placés à droite de ce même axe, sont placés dans l'état gonflé. La poche supérieure 43 se dégonfle progressivement, tandis que le fluide de culture se concentre dans la région située sensiblement au-dessus des groupes de membranes inférieures 46-2 et 46-3, dégonflés.

Dans la seconde étape, on maintient dans l'état fermé la vanne contrôlant l'accès à la sortie du conduit d'évacuation 56, tandis que l'on place dans l'état ouvert la vanne contrôlant l'accès à l'entrée du conduit d'alimentation 53. Dans le même temps, on ferme la vanne contrôlant l'entrée/sortie 59 de la poche supérieure déformable 43, et l'on place l'intégralité des membranes inférieures 46-i dans l'état dégonflé, permettant ainsi au fluide de culture de pénétrer à l'intérieur de la partie centrale 40 de la chambre de culture 6.

Un tel mode d'alimentation assure un remplacement maximal du milieu de culture. En diminuant le nombre de groupes de membranes inférieures participant au cycle d'alimentation, on peut gérer le volume de fluide échangé.

Dans ce qui précède, on a décrit des dispositifs dans lesquels l'accès aux entrées ou sorties de la chambre de culture était contrôlé par des vannes, et plus particulièrement par des électrovannes pilotées par les moyens de commande 3 et 4. Mais, tout autre type de moyens de contrôle d'accès pourrait être envisagé, et notamment des moyens comportant un conduit flexible à parois amincies logé dans un boîtier raccordé à un circuit de pressurisation permettant d'introduire dans ledit boîtier soit un gaz sous haute pression contraignant les parois amincies du conduit à se contacter, soit un gaz sous basse pression permettant à ces parois amincies d'être écartées l'une de l'autre et par conséquent de laisser passer le gaz ou le fluide à l'intérieur du conduit. Par ailleurs, le dispositif pourra comporter une multiplicité de membranes inférieures et une multiplicité de membranes supérieures. Par exemple, chaque multiplicité pourra être subdivisée en groupes et/ou en sous-groupes, le nombre de groupes et/ou sous-groupes de membranes inférieures n'étant pas forcément identique à celui des membranes supérieures. Dans le cas d'une identité de nombre, il pourra être avantageux de prévoir dans la partie centrale 40 des moyens de cloisonnement définissant dans celle-ci des cavités de culture s'étendant chacune d'un groupe de membranes inférieures 46-i à un groupe de membranes supérieures. Chaque cavité est dans ce cas agencée pour être alimentée en fluide de culture et/ou en gaz. A cet effet, il est avantageux que les moyens de cloisonnement comportent des cloisons réalisées dans un matériau poreux au fluide de culture et au gaz, mais imperméable aux cellules et tissus. La chambre de culture peut être ainsi subdivisée en une multiplicité de sous-chambres de culture propres à faire croître des cellules et/ou tissus différents ou non, en parallèle.

Par ailleurs, la première partie supérieure 17 pourra loger au moins une membrane latérale déformable définissant une interface entre une partie latérale et la partie centrale 40 contenant les cellules ou tissus. Dans ce cas, la partie latérale comprend une entrée alimentée en gaz par une sortie du circuit de pressurisation 2 et une sortie permettant d'évacuer une partie au moins de ce gaz hors de la chambre, et de préférence dans le circuit de pressurisation. Les moyens de commande 3,4 sont alors agencés pour déterminer, en fonction des critères choisis, la pression et la durée d'introduction du gaz, et pour contrôler les accès aux entrée et sortie de la partie latérale de manière à gérer la forme de la membrane latérale. Cela permet d'accroître encore le nombre de modes de circulation dans la chambre.

Le gaz introduit dans la partie latérale pourra être le gaz choisi qui alimente la partie inférieure 16, mais il pourrait également s'agir d'un autre gaz, moyennant les adaptations nécessaires de la chambre de culture et du circuit de pressurisation (deux branches d'alimentation parallèles couplées chacune à des vannes d'entrée et de sortie).

D'autre part, dans ce qui précède on a fait référence à un circuit de pressurisation de type fermé. Mais l'invention concerne également les circuits de pressurisation «ouverts» comprenant, par exemple un (ou plusieurs) réservoir(s) de gaz raccordé(s) via une (ou plusieurs) vanne(s) à l'entrée (ou aux entrées) de la partie inférieure 16 et/ou de la partie latérale et/ou de la partie arrière 41 de la chambre de culture. Dans ce cas, la (ou les) sortie(s) de la (ou des) partie(s) concemée(s) peu(ven)t communiquer directement avec l'extérieur, le (ou les) gaz assurant la pressurisation des membranes et/ou poche n'étant alors pas réutilisé(s) dans le circuit de pressurisation.

En résumé, le dispositif selon l'invention agit comme une pompe qui assure plusieurs fonctions en combinaison, et notamment un échange moléculaire au niveau des gaz, une agitation/circulation du fluide de culture et des cellules et/ou tissus, et un échange thermique entre le gaz et le fluide de culture.

L'invention ne se limite pas aux seuls dispositifs de culture de cellules et tissus qui viennent d'être décrits, à titre d'exemples non limitatifs. L'invention concerne en effet, également, un procédé de culture de cellules et tissus qui comporte deux étapes.

La première étape consiste à prévoir un dispositif de culture du type de celui décrit précédemment, et comprenant au moins une chambre de culture munie d'au moins une membrane déformable placée à l'interface entre des première et seconde parties à volumes complémentaires variables, la première partie (supérieure) pouvant être alimentée en fluide de culture et recevant les cellules et/ou tissus à cultiver, et la seconde partie (inférieure) étant alimentée en un gaz.

La seconde étape consiste à alimenter les première et seconde parties selon des pressions, débits et durées choisis en fonction de critères relatifs au type de culture à effectuer, de manière à faire varier la forme de la (ou des) membrane(s) pendant toute la durée de la culture, créant ainsi une circulation contrôlée du fluide de culture à l'intérieur de la première partie de la chambre.

De préférence, dans la première étape de ce procédé, la chambre ne comprend pas une unique membrane, mais une multiplicité de membranes indépendantes, celles-ci étant plus préférentiellement encore subdivisées en au moins deux groupes indépendants. Dans ce cas, il est particulièrement avantageux que lors de la seconde étape, chaque groupe soit alimenté en gaz choisi, indépendamment des autres groupes de sorte que les formes respectives des membranes de chaque groupe varient indépendamment. Dans cette première étape du procédé, on pourra également prévoir, à l'intérieur de la première partie de la chambre de culture, du côté opposé à la seconde partie, une poche déformable alimentée en gaz. Dans ce cas, lors de la seconde étape, la poche est alimentée selon des pressions, débits et durées choisis en fonction des critères de culture précités, de manière à faire varier la forme de la poche conjointement avec celle de chaque membrane, pendant la durée de la culture.

En variante, la poche déformable supérieure peut être remplacée par une ou plusieurs membranes auxiliaires déformables placées à l'interface entre une partie centrale recevant les cellules ou tissus à cultiver et une partie arrière alimentée en gaz. Par ailleurs, il est avantageux que lors de la première étape, la seconde partie et la poche ou la partie arrière soient alimentées par le même gaz choisi.

Il est également avantageux que chaque membrane et la poche ou la membrane auxiliaire soient réalisées dans un matériau poreux, au moins dans le sens allant vers la première partie (partie centrale), de sorte que le gaz choisi permettant de faire varier les formes des membranes et de la poche puisse pénétrer au moins partiellement dans la première partie (partie centrale) pour participer à la culture avec le fluide de culture.

Tous les modes de circulation qui ont été décrits dans la partie relative aux dispositifs selon l'invention doivent être également considérés comme des exemples de seconde étape du procédé selon l'invention. Notamment, le procédé permet, grâce à sa seconde étape, d'assurer un déplacement du fluide et/ou des cellules et tissus de manière à créer dans la chambre de culture des zones dans lesquelles les concentrations cellulaires sont différentes entre elles.

L'invention s'applique à de très nombreux types de cellules et tissus, tels que notamment :
- les cellules intestines : Intestine 407, Caco-2, Cola 205, T84, SW 1116, WiDr, HT 29, HT 115, HT 55 ;
- les cellules endothéliales : HAOSMC (de l'acronyme anglais Human Aortic Smooth Muscle Cells) ;
- les cellules épidermales: NHEK-Neopooled (Human Epidermal Keratinocyte Neonatal), Equine Dermis ;
- les cellules cancéreuses : HeLa, CHO-K1 ;
- les cellules fibroplastiques de type intestinales : CCD-18Co
- les cellules fibroplastiques de type MRC-5, 3T3, Wi-38 ;
- les myélomes : SP2O-Ag14, P3X63 Ag8 653, MPC11;
- les hybridomes ;
- les cellules d'insectes : SF9.

Bien entendu, certains de ces tissus ou certaines de ces cellules nécessitent l'utilisation d'un support de culture, comme exposé précédemment.

Cette liste n'est en aucun cas exhaustive ; il ne s'agit que d'exemples.

Le dispositif selon l'invention peut comporter des caractéristiques additionnelles à celles présentées ci-avant, et notamment :
- le circuit de pressurisation peut comporter au moins une entrée raccordée à un réservoir qui contient le gaz choisi et au moins une sortie qui alimente l'entrée de la seconde partie ;
- la poche peut être formée d'une membrane ;
- la première partie peut comprendre au moins une entrée propre à être alimentée en fluide de culture par le premier réservoir et au moins une sortie propre à évacuer le fluide de culture usagé dans un second réservoir, et les moyens de commande peuvent être agencés de manière à contrôler les accès aux différentes entrées et sorties des première et seconde parties de manière à gérer conjointement la forme de la membrane, l'alimentation en fluide de culture et la circulation de ce fluide dans la première partie. Dans ce cas, il est avantageux que le circuit de pressurisation soit de type fermé et comporte au moins une entrée propre à l'alimenter en gaz choisi, un compresseur, propre à délivrer le gaz choisi sous la pression choisie, et au moins une sortie alimentée par ce compresseur et propre à alimenter l'entrée de la seconde partie ;
- les moyens de support peuvent être agencés pour supporter une multiplicité de membranes indépendantes. Dans ce cas, la multiplicité peut être subdivisée en au moins deux groupes de membranes indépendants, la seconde partie comportant alors au moins deux entrées pour alimenter en gaz choisi chaque groupe de membranes, indépendamment, et les moyens de commande étant agencés pour contrôler l'accès à ces deux entrées de manière à gérer les formes respectives des membranes de chaque groupe. En variante, la multiplicité peut être subdivisée en quatre groupes de membranes indépendantes, la seconde partie comportant quatre entrées pour alimenter en gaz choisi chaque groupe de membranes, indépendamment, et les moyens de commande étant agencés pour contrôler l'accès à ces quatre entrées de manière à gérer les formes respectives des membranes de chaque groupe ;
- la chambre de culture peut loger dans la première partie, du côté opposé à la seconde partie, des moyens de support auxiliaires propres à supporter au moins une membrane auxiliaire déformable formant dans cette première partie une interface entre une partie centrale propre à recevoir les cellules ou tissus à cultiver et une partie arrière comportant une entrée propre à être alimentée en gaz par le circuit de pressurisation et une sortie raccordée à ce circuit de pressurisation, les moyens de commande étant agencés pour déterminer, en fonction des critères relatifs au type de culture à effectuer, la pression et la durée d'introduction de ce gaz, et pour contrôler les accès à l'entrée et à la sortie de la partie arrière de manière à gérer la forme de la membrane auxiliaire conjointement avec la forme de chaque membrane ;
- le circuit de pressurisation peut comporter une autre sortie propre à alimenter en gaz choisi, sous une autre pression choisie, l'entrée de la poche ou de la partie arrière ;
- l'entrée et la sortie de la poche ou de la partie arrière peuvent être confondues ;
- les moyens de support auxiliaire peuvent être agencés pour supporter une multiplicité de membranes auxiliaires indépendantes. Dans ce cas, il est avantageux que la multiplicité soit subdivisée en au moins deux groupes de membranes auxiliaires indépendants, la partie arrière comportant au moins deux entrées pour alimenter en gaz choisi chaque groupe de membranes auxiliaires, indépendamment, et les moyens de commande étant agencés pour contrôler l'accès à ces deux entrées de manière à gérer les formes respectives des membranes auxiliaires de chaque groupe ;
- l'entrée et la sortie de la seconde partie peuvent être confondues ;
- la chambre de culture pourra être délimitée par un boîtier comprenant une partie inférieure formant un réceptacle comportant une ouverture pour chaque entrée et pour chaque sortie des première et seconde parties de la chambre. Ce réceptacle peut comporter une ouverture pour chaque entrée et pour chaque sortie de la seconde partie de la chambre, le couvercle comprenant alors une ouverture adaptée à l'entrée et à la sortie de la poche ou de la partie arrière, et les moyens de support auxiliaire étant solidarisés au couvercle ;
- on peut prévoir au moins une sonde, choisie parmi au moins une sonde de pH et une sonde de température, raccordée aux moyens de commande et propre à être introduite à l'intérieur de la première partie de la chambre, le réceptacle comportant alors au moins une première ouverture auxiliaire adaptée à l'introduction de chaque sonde ;
- on peut prévoir deux entrées pilotées par les moyens de commande et propres à être alimentées respectivement en de premier et second gaz choisis ;
- chaque entrée et chaque sortie peuvent être contrôlées par un moyen de commande d'accès piloté par les moyens de commande ;
- les moyens de support peuvent comporter un bâti logé dans le réceptacle et de préférence réalisé dans un matériau synthétique, en particulier en élastomère ;
- la première partie peut loger au moins une membrane latérale déformable formant dans la première partie une interface entre une partie latérale et la partie contenant les cellules ou tissus, la partie latérale comprenant une entrée propre à être alimentée en gaz par le circuit de pressurisation et une sortie pour évacuer une partie au moins de ce gaz, et les moyens de commande étant agencés pour déterminer, en fonction des critères choisis, la pression et la durée d'introduction du gaz, et pour contrôler les accès aux entrée et sortie de la partie latérale de manière à gérer la forme de la membrane latérale. Dans ce cas, il est avantageux que le gaz introduit dans la partie latérale soit le gaz choisi ;
- l'un au moins des premier et second réservoirs peut comprendre au moins une poche-réservoir souple propre à alimenter en fluide de culture la première partie ou à recueillir le fluide usagé. Dans ce cas, il est avantageux qu'au moins le premier réservoir comprenne au moins deux poches-réservoir souples propre à alimenter en fluide de culture différents la première partie ;
- les moyens de commande et le circuit de pressurisation (2) peuvent être logés dans un premier compartiment, tandis qu'un second compartiment loge l'unité de culture. Dans ce cas, il est avantageux que le second compartiment loge au moins une autre unité de culture, et que le circuit de pressurisation comprenne au moins une première partie de dérivation, raccordée à chacune de ses sorties qui délivrent le gaz sous pression choisie, de manière à alimenter en gaz au moins l'autre unité de culture ;
- le circuit de pressurisation peut comprendre une seconde partie de dérivation, raccordée à chacune de ses sorties délivrant le gaz sous pression choisie, de manière à alimenter en gaz au moins une unité de culture externe ;
- le premier compartiment peut comprendre un sous-compartiment formant un réservoir de gaz alimenté par chaque entrée d'alimentation en gaz choisi et alimentant le compresseur, et pouvant loger un capteur de température agencé pour délivrer des mesures de température aux moyens de commande ainsi qu'un moyen de chauffage du gaz piloté par les moyens de contrôle en fonction des critères choisis et des mesures de température. Il est également avantageux que le sous-compartiment loge un analyseur de gaz agencé pour délivrer aux moyens de commande des mesures représentatives du pourcentage d'au moins une espèce de molécules à l'intérieur du sous-compartiment, et que les moyens de commande soient agencés pour contrôler l'accès aux différentes entrées et sorties en fonction des critères choisis et des mesures de pourcentage. Par ailleurs, le circuit de pressurisation peut comporter un conduit reliant au moins une sortie du sous-compartiment à l'entrée de la poche ou de la partie arrière de la première partie. D'autre part, le sous-compartiment peut être subdivisé en au moins de première et seconde sous-parties étanches formant respectivement un premier réservoir de gaz basse pression, alimenté par chaque entrée d'alimentation en gaz choisi et alimentant le compresseur, et un second réservoir de gaz haute pression alimenté par le compresseur et alimentant chaque sortie du circuit de pressurisation en gaz choisi. Dans ce cas, il est avantageux que la première sous-partie du sous-compartiment loge l'analyseur de gaz et la seconde sous-partie du sous-compartiment loge le capteur de température et le moyen de chauffage du gaz.

De même, le procédé selon l'invention peut comporter des caractéristiques additionnelles à celles présentées ci-avant, et notamment :
- dans la seconde étape les différences de concentrations cellulaires peuvent être obtenues par des concentrations cellulaires initiales différentes dans les zones de culture et/ou des cycles de déformation de membrane(s) et/ou poche qui diffèrent d'une zone à l'autre;
- la seconde étape peut être propre à assurer conjointement un contrôle de la pression et de la circulation du fluide de culture dans la première partie de la chambre ;
- dans la première étape on peut prévoir, dans la première partie, des moyens de cloisonnement définissant des cavités de culture qui s'étendent chacune d'un groupe de membranes à un groupe de membranes auxiliaires, et propres chacune à être alimentées en fluide de culture et à recevoir des cellules adhérentes ainsi que des supports de culture et/ou des cellules non adhérentes et/ou des tissus, éventuellement différents d'une cavité à l'autre.

## Revendications

1. Dispositif de culture cellulaire et tissulaire à circulation de fluide de culture contrôlée, **caractérisé en ce qu'**il comprend :
- un circuit de pressurisation (2) agencé pour délivrer au moins un gaz choisi, sous une pression choisie,
- au moins une chambre de culture (6) logeant des moyens (13) propres à supporter au moins une membrane (14 ;46-i) déformable formant dans la chambre (6) une interface entre de première (17) et seconde (16) parties à volumes complémentaires variables, ladite première partie (17) étant agencée pour recevoir des cellules et/ou des tissus à cultiver et pour être alimentée en fluide de culture par un premier réservoir (8), et ladite seconde partie (16) comprenant une entrée (18 ;47-i) propre à être alimentée en gaz par ledit circuit de pressurisation (2) et une sortie (18 ;47-i) pour évacuer une partie au moins de ce gaz, et
- des moyens de commande (3,4) agencés pour déterminer, en fonction de critères choisis relatifs au type de culture à effectuer, le gaz à introduire dans la seconde partie (16), sa pression et la durée d'introduction de ce gaz, et pour contrôler les accès aux première (17) et seconde (16) parties de la chambre (6) de manière à gérer conjointement la forme de la membrane (14 ;46-i), l'alimentation en fluide de culture et la circulation de ce fluide dans la première partie (17).

2. Dispositif selon la revendication1, **caractérisé en ce qu'**il comporte une sur-membrane (90) placée au-dessus de la membrane (14), du côté de la première partie (17) et délimitant avec cette membrane (14) au moins une zone intermédiaire (91), **en ce que** ladite chambre (6) comprend au moins une entrée (93) propre à être alimentée en gaz par le circuit de pressurisation (2) et à alimenter en ledit gaz ladite zone intermédiaire (91) et au moins une sortie (94) pour évacuer de cette zone intermédiaire (91) une partie au moins du gaz, et **en ce que** lesdits moyens de commande (3,4) sont agencés pour déterminer, en fonction des critères choisis, le gaz à introduire dans la zone intermédiaire (91), sa pression et la durée d'introduction de ce gaz, et pour contrôler les accès aux entrée (93) et sortie (94) de cette zone intermédiaire (91) de manière à gérer conjointement les formes de la membrane (14 ;46-i) et de la sur-membrane (90) et la circulation du fluide de culture dans la première partie (17).

3. Dispositif selon la revendication 2, **caractérisé en ce que** la sur-membrane (90) est solidarisée à la membrane (14) en une multiplicité d'endroits choisis, de manière à subdiviser la zone intermédiaire (91) en une pluralité d'alvéoles (92) communicantes à volumes variables.

4. Dispositif selon l'une des revendications 1 à 3, **caractérisé en ce que** ledit circuit de pressurisation (2) comporte au moins une entrée raccordée à un réservoir contenant ledit gaz choisi et au moins une sortie (99) alimentant l'entrée (18 ;47-i) de la seconde partie (16).

5. Dispositif selon l'une des revendications 1 à 4, **caractérisé en ce que** ladite première partie (17) comprend au moins une entrée (19) propre à être alimentée en fluide de culture par ledit premier réservoir (8) et au moins une sortie (20) propre à évacuer le fluide de culture usagé dans un second réservoir (9), et **en ce que** lesdits moyens de commande (3,4) sont agencés pour contrôler les accès aux différentes entrées et sorties des première (17) et seconde (16) parties de manière à gérer conjointement la forme de la membrane (14 ;46-i), l'alimentation en fluide de culture et la circulation de ce fluide dans la première partie (17).

6. Dispositif selon l'une des revendications 1 à 3 en combinaison avec la revendication 5, **caractérisé en ce que** ledit circuit de pressurisation (2) est de type fermé et comporte au moins une entrée (26,27) propre à l'alimenter en gaz choisi, un compresseur (30) propre à délivrer le gaz choisi sous la pression choisie et au moins une sortie (99) alimentée par ledit compresseur (30) et propre à alimenter l'entrée (18 ; 47-i) de la seconde partie (16).

7. Dispositif selon l'une des revendications 1 à 6, **caractérisé en ce que** lesdits moyens de support (13) sont agencés pour supporter une multiplicité de membranes indépendantes (46-i).

8. Dispositif selon la revendication 7, **caractérisé en ce que** ladite multiplicité est subdivisée en au moins deux groupes de membranes indépendants, **en ce que** ladite seconde partie (16) comporte au moins deux entrées (47-i) pour alimenter en gaz choisi chaque groupe de membranes (46-i), indépendamment, et **en ce que** lesdits moyens de commande (3,4) sont agencés pour contrôler l'accès à ces deux entrées (48-i) de manière à gérer les formes respectives des membranes de chaque groupe.

9. Dispositif selon la revendication 7, **caractérisé en ce que** ladite multiplicité est subdivisée en quatre groupes de membranes indépendantes, **en ce que** ladite seconde partie (16) comporte quatre entrées (47-i) pour alimenter en gaz choisi chaque groupe de membranes, indépendamment, et **en ce que** lesdits moyens de commande (3,4) sont agencés pour contrôler l'accès à ces quatre entrées de manière à gérer les formes respectives des membranes de chaque groupe (46-i).

10. Dispositif selon l'une des revendications 1 à 9, **caractérisé en ce que** ladite chambre de culture (6) loge dans la première partie (17), du côté opposé à la seconde partie (16), une poche déformable (43) munie d'une entrée (59) alimentée en gaz par le circuit de pressurisation (2) et d'une sortie (59) raccordée à ce circuit de pressurisation (2), et **en ce que** lesdits moyens de commande (3,4) sont agencés pour déterminer, en fonction desdits critères relatifs au type de culture à effectuer, la pression et la durée d'introduction de ce gaz, et pour contrôler les accès à l'entrée et à la sortie de la poche (43) de manière à gérer sa forme conjointement avec la forme de chaque membrane (46-i).

11. Dispositif selon la revendication 10, **caractérisé en ce que** la poche (43) est formée d'une membrane.

12. Dispositif selon l'une des revendications 1 à 9, **caractérisé en ce que** ladite chambre de culture (6) loge dans la première partie (17), du côté opposé à la seconde partie (16), des moyens de support auxiliaires (12) propres à supporter au moins une membrane auxiliaire déformable (39) formant dans la première partie (17) une interface entre une partie centrale (40) propre à recevoir les cellules ou tissus à cultiver et une partie arrière (41) comportant une entrée (42) propre à être alimentée en gaz par le circuit de pressurisation (2) et une sortie (42) raccordée à ce circuit de pressurisation (2), et **en ce que** lesdits moyens de commande (3,4) sont agencés pour déterminer, en fonction desdits critères relatifs au type de culture à effectuer, la pression et la durée d'introduction de ce gaz, et pour contrôler les accès à l'entrée et à la sortie de la partie arrière (41) de manière à gérer la forme de la membrane auxiliaire (39) conjointement avec la forme de chaque membrane (14 ;46-i).

13. Dispositif selon l'une des revendications 10 à 12, **caractérisé en ce que** ledit circuit de pressurisation (2) comporte une autre sortie (100) propre à alimenter en ledit gaz choisi, sous une autre pression choisie, l'entrée (59 ;42) de la poche (43) ou de la partie arrière (41).

14. Dispositif selon l'une des revendications 10 à 13, **caractérisé en ce que** l'entrée et la sortie de la poche (43) ou de la partie arrière (41) sont confondues.

15. Dispositif selon l'une des revendications 10 à 14, **caractérisé en ce que** lesdits moyens de support auxiliaire (12) sont agencés pour supporter une multiplicité de membranes auxiliaires indépendantes.

16. Dispositif selon la revendication 15, **caractérisé en ce que** ladite multiplicité est subdivisée en au moins deux groupes de membranes auxiliaires indépendants, **en ce que** ladite partie arrière (41) comporte au moins deux entrées pour alimenter en gaz choisi chaque groupe de membranes auxiliaires, indépendamment, et **en ce que** lesdits moyens de commande (3,4) sont agencés pour contrôler l'accès à ces deux entrées de manière à gérer les formes respectives des membranes auxiliaires de chaque groupe.

17. Dispositif selon la revendication 15, **caractérisé en ce que** ladite multiplicité est subdivisée en quatre groupes de membranes auxiliaires indépendantes, **en ce que** ladite partie arrière (41) comporte quatre entrées pour alimenter en gaz choisi chaque groupe de membranes auxiliaires, indépendamment, et **en ce que** lesdits moyens de commande (3,4) sont agencés pour contrôler l'accès à ces quatre entrées de manière à gérer les formes respectives des membranes auxiliaires de chaque groupe.

18. Dispositif selon l'une des revendications 8 et 9 en combinaison avec l'une des revendications 16 et 17, **caractérisé en ce que** la partie centrale (40) de la première partie (17) loge des moyens de cloisonnement définissant dans celle-ci des cavités de culture s'étendant chacune d'un groupe de membranes (46-i) à un groupe de membranes auxiliaires, chaque cavité étant propre à être alimentée en fluide de culture.

19. Dispositif selon la revendication 18, **caractérisé en ce que** lesdits moyens de cloisonnement comportent des cloisons réalisées dans un matériau poreux audit fluide de culture, mais imperméable aux cellules et tissus, de sorte que ledit fluide de culture puisse circuler d'une cavité à l'autre.

20. Dispositif selon l'une des revendications 1 à 19, **caractérisé en ce que** la première partie (17) comporte au moins une ouverture munie d'un moyen étanche, en particulier de type septum, propre à permettre l'introduction ou l'extraction de cellule ou tissus.

21. Dispositif selon l'une des revendications 1 à 20, **caractérisé en ce que** l'entrée et la sortie (18 ;47-i) de la seconde partie (16) sont confondues.

22. Dispositif selon l'une des revendications 1 à 21, **caractérisé en ce que** lesdites membranes (14 ;39 ;43 ;46-i) et membranes auxiliaires sont réalisées dans un matériau poreux, au moins dans le sens allant vers la première partie (17), de sorte que le gaz choisi, introduit dans la seconde partie (16), puisse pénétrer au moins partiellement dans ladite première partie (17).

23. Dispositif selon l'une des revendications 5 à 22, **caractérisé en ce que** ladite chambre de culture (6) est délimitée par un boîtier comprenant une partie inférieure formant réceptacle (11), comportant au moins une ouverture adaptée pour l'entrée (19) d'alimentation en fluide de culture, la sortie (20) d'évacuation du fluide de culture usé, l'entrée (18 ;46-i) (ou les entrées) d'alimentation en gaz choisi de la seconde partie (16) et la (ou les) sortie(s) (18 ;22) raccordée(s) au circuit de pressurisation (2), une partie supérieure formant couvercle (12), et **en ce que** lesdits moyens de support comprennent un bâti (44) logé dans le réceptacle (11) et dans lequel se trouvent définies des cavités (45-i) propres chacune à supporter à étanchéité une membrane (46-i), à être alimentées en ledit gaz choisi et à loger ladite membrane lorsque la pression du gaz choisi est inférieure à un premier seuil.

24. Dispositif selon la revendication 23, **caractérisé en ce que** ledit couvercle (12) est réalisé dans un matériau transparent.

25. Dispositif selon l'une des revendications 23 et 24, **caractérisé en ce que** ledit réceptacle (11) comporte une ouverture pour chaque entrée et pour chaque sortie des première (17) et seconde (16) parties de la chambre (6).

26. Dispositif selon l'une des revendications 23 et 24 en combinaison avec l'une des revendications 10 et 12, **caractérisé en ce que** ledit réceptacle (11) comporte une ouverture pour chaque entrée et pour chaque sortie de la seconde partie (16) de la chambre (6), **en ce que** ledit couvercle (12) comprend une ouverture adaptée à l'entrée et à la sortie (42 ;59) de la poche (43) ou de la partie arrière (41), et **en ce que** lesdits moyens de support auxiliaire sont solidarisés audit couvercle (12).

27. Dispositif selon l'une des revendications 23 à 26, **caractérisé en ce qu'**il comprend au moins une sonde, choisie parmi au moins une sonde de pH (37) et une sonde de température (36), raccordée auxdits moyens de commande (3) et propre à être introduite à l'intérieur de la première partie (17) de la chambre (6), et **en ce que** ledit réceptacle (11) comporte au moins une première ouverture auxiliaire (52,51) adaptée à l'introduction de chaque sonde (36,37).

28. Dispositif selon l'une des revendications 1 à 27, **caractérisé en ce qu'**il comprend deux entrées (26,27) pilotées par lesdits moyens de commande (3,4) et propres à être alimentées respectivement en de premier et second gaz choisis.

29. Dispositif selon l'une des revendications 1 à 28, **caractérisé en ce que** chaque entrée et chaque sortie sont contrôlées par un moyen de commande d'accès (28 ;29 ;32 ;49-i ;75) piloté par lesdits moyens de commande (3,4).

30. Dispositif selon l'une des revendications 23 à 29, **caractérisé en ce que** ledit bâti (44) est réalisé dans un matériau synthétique, en particulier en élastomère.

31. Dispositif selon l'une des revendications 1 à 30, **caractérisé en ce que** ladite première partie (17) loge au moins une membrane latérale déformable formant dans la première partie (17) une interface entre une partie latérale et la partie (41) contenant les cellules ou tissus, ladite partie latérale comprenant une entrée propre à être alimentée en gaz par ledit circuit de pressurisation (2) et une sortie pour évacuer une partie au moins de ce gaz, et **en ce que** les moyens de commande (3,4) sont agencés pour déterminer, en fonction des critères choisis, la pression et la durée d'introduction du gaz, et pour contrôler les accès aux entrée et sortie de la partie latérale de manière à gérer la forme de la membrane latérale.

32. Dispositif selon la revendication 31, **caractérisé en ce que** ledit gaz introduit dans ladite partie latérale est le gaz choisi.

33. Dispositif selon l'une des revendications 1 à 32, **caractérisé en ce qu'**il comprend au moins une unité de culture (7) comportant le premier réservoir (8) raccordé à la chambre de culture (6) et un second réservoir (9) raccordé à cette chambre de culture (6) de manière à recueillir le fluide de culture usé.

34. Dispositif selon la revendication 33, **caractérisé en ce que** chaque premier (8) et second (9) réservoir comprend au moins une ouverture munie d'un moyen étanche (65), en particulier de type septum, propre à permettre l'introduction ou l'extraction de fluide.

35. Dispositif selon l'une des revendications 1 à 34, **caractérisé en ce que** l'un au moins des premier (8) et second (9) réservoir comprend au moins une poche-réservoir souple propre à alimenter en fluide de culture ladite première partie (17) ou à recueillir le fluide usagé.

36. Dispositif selon la revendication 35, **caractérisé en ce qu'**au moins le premier réservoir (8) comprend au moins deux poches-réservoir souples propre à alimenter en fluide de culture différents ladite première partie (17).

37. Dispositif selon l'une des revendications 33 à 36, **caractérisé en ce qu'**il comprend un premier compartiment (1) logeant les moyens de commande (3,4) et le circuit de pressurisation (2), et un second compartiment (5) logeant ladite unité de culture (7).

38. Dispositif selon la revendication 37, **caractérisé en ce que** ledit second compartiment (5) loge au moins une autre unité de culture, et **en ce que** ledit circuit de pressurisation (2) comprend au moins une première partie de dérivation, raccordée à chacune de ses sorties (99) délivrant ledit gaz sous pression choisie, de manière à alimenter en gaz au moins l'autre unité de culture.

39. Dispositif selon l'une des revendications 33 à 38, **caractérisé en ce que** ledit circuit de pressurisation (2) comprend une seconde partie de dérivation (62), raccordée à chacune de ses sorties (99) délivrant ledit gaz sous pression choisie, de manière à alimenter en gaz au moins une unité de culture externe.

40. Dispositif selon l'une des revendications 37 à 39, **caractérisé en ce que** ledit premier compartiment (1) comprend un sous-compartiment (25) formant réservoir de gaz alimenté par chaque entrée (26 ;27) d'alimentation en gaz choisi et alimentant ledit compresseur (30).

41. Dispositif selon la revendication 40, **caractérisé en ce que** ledit sous-compartiment (25) loge un capteur de température (34) agencé pour délivrer des mesures de température aux moyens de commande (3,4) et un moyen de chauffage du gaz (33) piloté par lesdits moyens de contrôle (3,4) en fonction desdits critères choisis et desdites mesures de température.

42. Dispositif selon l'une des revendications 40 et 41, **caractérisé en ce que** ledit sous-compartiment (25) loge un analyseur de gaz (35) agencé pour délivrer aux moyens de commande (3,4) des mesures représentatives du pourcentage d'au moins une espèce de molécules à l'intérieur dudit sous-compartiment (25), et **en ce que** lesdits moyens de commande (3,4) sont agencés pour contrôler l'accès aux différentes entrées et sorties en fonction desdits critères choisis et desdites mesures de pourcentage.

43. Dispositif selon l'une des revendications 40 à 42 en combinaison avec l'une des revendications 10 et 12, **caractérisé en ce que** ledit circuit de pressurisation (2) comporte un conduit (100) reliant au moins une sortie (74) dudit sous-compartiment (25) à l'entrée (59 ;42) de ladite poche (43) ou de ladite partie arrière (41) de la première partie (17).

44. Dispositif selon l'une des revendications 40 à 43, **caractérisé en ce que** ledit sous-compartiment (25) est subdivisé en au moins de première (67) et seconde (69) sous-parties étanches formant respectivement un premier réservoir de gaz basse pression, alimenté par chaque entrée (26 ;27) d'alimentation en gaz choisi et alimentant ledit compresseur (30), et un second réservoir de gaz haute pression alimenté par ledit compresseur (30) et alimentant chaque sortie (99,62) du circuit de pressurisation (2) en gaz choisi.

45. Dispositif selon la revendication 44 en combinaison avec les revendications 41 et 42, **caractérisé en ce que** la première sous-partie (67) du sous-compartiment (25) loge ledit analyseur de gaz (35) et la seconde sous-partie (69) du sous-compartiment (25) loge ledit capteur de température (34) et ledit moyen de chauffage du gaz (33).

46. Dispositif selon l'une des revendications 44 et 45, **caractérisé en ce que** ledit sous-compartiment (25) est subdivisé en première (67), seconde (69) et troisième (68) sous-parties étanches, ladite troisième (68) sous-partie étant placée entre les première (67) et seconde (69) sous-parties et formant un troisième réservoir de gaz de pression intermédiaire, lesdites sous-parties communicant entre elles par une ouverture à accès contrôlé par lesdits moyens de commande (3,4).

47. Dispositif selon l'une des revendications 44 à 46, **caractérisé en ce que** chaque sous-partie (67-69) alimente en gaz choisi, sous contrôle de moyens de contrôle d'accès (49-i) pilotés par lesdits moyens de commande, chaque sortie (100,48-i) du circuit de pressurisation, délivrant ledit gaz choisi à ladite chambre de culture.

48. Procédé de culture cellulaire et tissulaire à circulation de fluide de culture contrôlée, **caractérisé en ce qu'**il comprend :
- une première étape dans laquelle on prévoit un dispositif de culture comprenant au moins une chambre de culture munie d'au moins une membrane déformable à l'interface entre de première et seconde parties à volumes complémentaires variables, ladite première partie étant propre à être alimentée en un fluide de culture et à recevoir des cellules et/ou des tissus à cultiver, et ladite seconde partie étant propre à être alimentée en un gaz ; et
- une seconde étape dans laquelle on alimente lesdites première et seconde parties selon des pressions, débits et durées choisis en fonction de critères relatifs au type de culture à effectuer, pour faire varier la forme de cette membrane pendant la durée de la culture, de manière à créer une circulation contrôlée du fluide de culture dans la première partie de la chambre.

49. Procédé selon la revendication 48, **caractérisé en ce qu'**à la première étape on prévoit une chambre comprenant une multiplicité de membranes indépendantes.

50. Procédé selon la revendication 49, **caractérisé en ce qu'**à la première étape on prévoit une subdivision de la multiplicité en au moins deux groupes de membranes indépendants, et **en ce qu'**à la seconde étape on alimente indépendamment en gaz choisi chaque groupe, de manière à faire varier indépendamment les formes respectives des membranes de chaque groupe.

51. Procédé selon la revendication 50, **caractérisé en ce qu'**à la première étape on prévoit une subdivision de la multiplicité en au moins quatre groupes de membranes indépendants, et **en ce qu'**à la seconde étape on alimente indépendamment en gaz choisi chaque groupe, de manière à faire varier indépendamment les formes respectives des membranes de chaque groupe.

52. Procédé selon l'une des revendications 49 à 51, **caractérisé en ce qu'**à la première étape on prévoit dans la première partie de la chambre de culture, du côté opposé à la seconde partie, une poche déformable alimentée en gaz, et **en ce qu'**à la seconde étape on alimente ladite poche selon des pressions, débits et durées choisis en fonction desdits critères, pour faire varier la forme de cette poche conjointement avec celle de chaque membrane pendant la durée de la culture, de manière à créer la circulation du fluide de culture.

53. Procédé selon l'une des revendications 49 à 51, **caractérisé en ce qu'**à la première étape on prévoit dans la première partie de la chambre de culture, du côté opposé à la seconde partie, au moins une membrane auxiliaire déformable formant interface entre une partie centrale recevant les cellules ou tissus à cultiver et une partie arrière alimentée en gaz, et **en ce qu'**à la seconde étape on alimente ladite partie arrière selon des pressions, débits et durées choisis en fonction desdits critères, pour faire varier la forme de la membrane auxiliaire conjointement avec celle de chaque membrane pendant la durée de la culture, de manière à créer la circulation de fluide de culture.

54. Procédé selon l'une des revendications 52 et 53, **caractérisé en ce qu'**à la première étape la seconde partie et la poche ou la partie arrière sont alimentées en le même gaz choisi.

55. Procédé selon l'une des revendications 49 à 54, **caractérisé en ce qu'**à la première étape chaque membrane et la poche ou la membrane auxiliaire sont réalisées dans un matériaux poreux, au moins dans le sens allant vers ta première partie, de sorte que le gaz choisi, introduit dans la seconde partie et/ou la poche ou la membrane auxiliaire, puisse pénétrer au moins partiellement dans ladite première partie.

56. Procédé selon l'une des revendications 48 à 55, **caractérisé en ce que** la seconde étape est propre à assurer un déplacement du fluide et/ou des cellules et tissus de manière à créer dans la chambre de culture des zones dans lesquelles les concentrations cellulaires sont différentes entre elles.

57. Procédé selon la revendication 56, **caractérisé en ce que** dans la seconde étape les différences de concentrations cellulaires s'effectuent par des modifications locales du volume de culture de la première partie.

58. Procédé selon la revendication 56, **caractérisé en ce que** dans la seconde étape les différences de concentrations cellulaires sont obtenues par des concentrations cellulaires initiales différentes dans lesdites zones de culture et/ou des cycles de déformation de membrane(s) et/ou poche qui diffèrent d'une zone à l'autre.

59. Procédé selon l'une des revendications 48 à 58, **caractérisé en ce que** la seconde étape est propre à assurer conjointement un contrôle de la pression et de la circulation du fluide de culture dans la première partie de la chambre.

60. Procédé selon l'une des revendications 53 à 59, **caractérisé en ce que** dans la première étape on prévoit dans la première partie des moyens de cloisonnement définissant des cavités de culture s'étendant chacune d'un groupe de membranes à un groupe de membranes auxiliaires, et propres chacune à être alimentées en fluide de culture et à recevoir des cellules adhérentes ainsi que des supports de culture et/ou des cellules non adhérentes et/ou des tissus, éventuellement différents d'une cavité à l'autre.

## Claims

1. A cell and tissue culture device with controlled culture fluid circulation, **characterized in that** it comprises:
- a pressurisation circuit (2) configured so as to deliver at least a selected gas under a selected pressure,
- at least a culture chamber (6) designed to receive appropriate means (13) for supporting at least a deformable membrane (14; 46-i) forming within the chamber (6) an interface between first (17) and second (16) portions with variable complementary volumes, said first portion (17) being configured for receiving cells and/or tissues to be grown and for being supplied with culture fluid from a first tank (8), and said second portion (16) comprising an inlet (18; 47-i) designed to be supplied with gas from said pressurisation circuit (2) and an outlet (18; 47-i) for discharging at least a portion of this gas, and
- control means (3, 4) designed to determine the gas to be fed into the second portion (16) according to selected criteria related to the type of culture to be achieved, its pressure and feed time of this gas, and to control access to first (17) and second (16) portions of the chamber (6) so as to control together the shape of the membrane (14; 46-i), the culture fluid supply and the flow of this fluid in the first portion (17).

2. A device according to claim 1, **characterized in that** it comprises a supramembrane (90) placed above the membrane (14), on the side of the first portion (17) and delimiting with this membrane (14) at least an intermediate area (91), **in that** said chamber (6) comprises at least an inlet (93) designed to be supplied with gas through the pressurisation circuit (2) and to supply with said gas said intermediate area (91) and at least an outlet (94) for discharging from this intermediate area (91) at least a portion of the gas, and **in that** said control means (3, 4) is designed to determine the gas to fed into the intermediate area (91) according to selected criteria, its pressure and the time for feeding in this gas, and to control access to inlet (93) and outlet (94) of this intermediate area (91) so as to control together the shapes of the membrane (14; 46-i) and supramembrane (90) and the flow of the culture fluid in the first portion (17).

3. A device according to claim 2, **characterized in that** the supramembrane (90) is firmly secured to membrane (14) in a multiplicity of selected locations, so as to subdivide the intermediate area (91) into a plurality of alveolar cavities (92) communicating with each other with variable volumes.

4. A device according to any of claims 1 to 3, **characterized in that** said pressurisation circuit (2) includes at least one inlet connected to a tank containing said selected gas and at least one outlet (99) feeding the inlet (18; 47-i) of the second portion (16).

5. A device according to any of claims 1 to 4, **characterized in that** said first portion (17) comprises at least one inlet (19) designed to be supplied with culture fluid by said first tank (8) and at least one outlet (20) designed to discharge waste culture fluid into a second tank (9), and **in that** said control means (3, 4) are configured in order to control access to the different inlets and outlets of first (17) and second (16) portions so as to control together the shape of the membrane (14; 46-i), the culture fluid supply and the flow of this fluid in the first portion (17).

6. A device according to any of claims 1 to 3, combined with claim 5, **characterized in that** said pressurisation circuit (2) is of the closed type and includes at least an inlet (26, 27) designed to supply it with a selected gas, a compressor (30) designed to deliver the selected gas under the selected pressure and at least an outlet (99) fed by said compressor (30) and designed to feed inlet (18; 47-i) of the second portion (16).

7. A device according to any of claims 1 to 6, **characterized in that** said supporting means (13) are designed to support a multiplicity of independent membranes (46-i).

8. A device according to claim 7, **characterized in that** said multiplicity is subdivided into at least two groups of independent membranes, **in that** said second portion (16) includes at least two inlets (47-i) for supplying with a selected gas each group of membranes (46-i), independently, and **in that** said control means (3, 4) are designed to control access to both these inlets (48-i) so as to control the respective shapes of the membranes of each group.

9. A device according to claim 7, **characterized in that** said multiplicity is subdivided into four groups of independent membranes, **in that** said second portion (16) comprises four inlets (47-i) for supplying with selected gas each group of membranes, independently, and **in that** said control means (3, 4) are designed to control access to these four inlets so as to control the respective shapes of the membranes of each group (46-i).

10. A device according to any of claims 1 to 9, **characterized in that** said culture chamber (6) contains in the first portion (17), on the opposite side to the second portion (16), a deformable pocket (43) which is provided with an inlet (59) supplied with gas by the pressurisation circuit (2) and an outlet (59) connected to this pressurisation circuit (2), and **in that** said control means (3, 4) are designed to determine the pressure and the time for feeding this gas according to said criteria related to the type of culture to be achieved, and to control access to the inlet and to the outlet of the pocket (43) so as to control its shape together with shape of each membrane (46-i).

11. A device according to claim 10, **characterized in that** the pocket (43) comprises a membrane.

12. A device according to any of claims 1 to 9, **characterized in that** said culture chamber (6) is designed to receive in the first portion (17), on the opposite side to the second portion (16), auxiliary supporting means (12) designed to support at least a deformable auxiliary membrane (39) which forms, in the first portion (17), an interface between a central portion (40) designed to receive cells or tissues to be grown and a rear portion (41) comprising an inlet (42) designed to be supplied with gas by the pressurisation circuit (2) and an outlet (42) connected- to this pressurisation circuit (2), and **in that** said control means (3, 4) are designed to determine the pressure and the time for feeding this gas according to said criteria related to the type of culture to be achieved, and to control access to the inlet and to the outlet of the rear portion (41) so as to control the shape of the auxiliary membrane (39) together with the shape of each membrane (14; 46-i).

13. A device according to any of claims 10 to 12, **characterized in that** said pressurisation circuit (2) comprises another outlet (100) designed to supply with said selected gas, under another selected pressure, the inlet (59; 42) of the pocket (43) or of the rear portion (41).

14. A device according to any of claims 10 to 13, **characterized in that** the inlet and the outlet of the pocket (43) or the rear portion (41) are merged.

15. A device according to any of claims 10 to 14, **characterized in that** said auxiliary supporting means (12) are designed to support a multiplicity of independent auxiliary membranes.

16. A device according to claim 15, **characterized in that** said multiplicity is subdivided into at least two groups of independent auxiliary membranes, **in that** said rear part (41) comprises at least two inlets for supplying each group of auxiliary membranes with selected gas, independently, and **in that** said control means (3, 4) are designed to control access to both these inlets so as to control the respective shapes of the auxiliary membranes of each group.

17. A device according to claim 15, **characterized in that** said multiplicity is subdivided into four groups of independent auxiliary membranes, **in that** said rear part (41) comprises four inlets for supplying each group of auxiliary membranes with selected gas, independently, and **in that** said control means (3, 4) are designed to control access to these four inlets so as to control the respective shapes of the auxiliary membranes of each group.

18. A device according to any of claims 8 and 9, combined with any of claims 16 and 17, **characterized in that** the central portion (40) of the first portion (17) is designed to receive partitioning means defining in the latter culture cavities each extending from a group of membranes (46-i) to a group of auxiliary membranes, each cavity being designed to be supplied with culture fluid.

19. A device according to claim 18, **characterized in that** said partitioning means comprises partitions made of a porous material to said culture fluid, but impervious to cells and tissues, so that said culture fluid may flow from one cavity to another.

20. A device according to any of claims 1 to 19, **characterized in that** the first portion (17) comprises at least a port provided with a sealing means, in particular of the septum type, enabling the introduction or extraction of cells or tissues.

21. A device according to any of claims 1 to 20, **characterized in that** the inlet and the outlet (18; 47-i) of the second portion (16) are merged.

22. A device according to any of claims 1 to 21, **characterized in that** said membranes (14; 39; 43; 46-i) and auxiliary membranes are made of a porous material, at least in the direction pointing to the first portion (17), so that the selected gas, fed into the second portion (16), at least partially penetrate said first portion (17).

23. A device according to any of claims 5 to 22, **characterized in that** said culture chamber (6) is delimited by a case comprising a lower portion forming a receptacle (11), including at least a port designed for the supply inlet (19) of culture fluid, the outlet (20) for discharging the waste culture fluid, the inlet (18; 46-i) (or inlets) for supplying the second portion (16) with the selected gas and the outlet(s) (18; 22) connected to the pressurisation circuit (2), an upper portion forming a lid (12), and **in that** said supporting means comprises a frame (44) arranged in the receptacle (11) and wherein cavities (45-i) are defined each designed to sealably support a membrane (46-i), to be supplied with said selected gas and to receive said membrane when the pressure of the selected gas is below a first threshold.

24. A device according to claim 23, **characterized in that** said lid (12) is made of a transparent material.

25. A device according to any of claims 23 and 24, **characterized in that** said receptacle (11) includes a port for each inlet and for each outlet of the first (17) and second (16) portions of the chamber (6).

26. A device according to any of claims 23 and 24 combined with any of claims 10 and 12, **characterized in that** said receptacle (11) includes a port for each inlet and for each outlet of the second portion (16) of chamber (6), **in that** said lid (12) comprises an adapted port for the inlet and for the outlet (42; 59) of pocket (43) or of the rear portion (41), and **in that** said auxiliary supporting means are firmly secured to said lid (12).

27. A device according to any of claims 23 to 26, **characterized in that** it comprises at least a probe, selected from at least a pH probe (37) and a temperature probe (36), connected to said control means (3) and designed to be introduced inside the first portion (17) of chamber (6), and **in that** said receptacle (11) includes at least an adapted first auxiliary port (52, 51) designed for the introduction of each probe (36, 37).

28. A device according to any of claims 1 to 27, **characterized in that** it comprises two inlets (26,27) driven by said control means (3, 4) and designed to be supplied with the first and second selected gas, respectively.

29. A device according to any of claims 1 to 28, **characterized in that** each inlet and each outlet is controlled by an access control means (28; 29; 32; 49-i; 75) driven by said control means (3, 4).

30. A device according to any of claims 23 to 29, **characterized in that** said frame (44) is made of a synthetic material, in particular, an elastomer.

31. A device according to any of claims 1 to 30, **characterized in that** said first portion (17) is designed to receive at least a deformable side membrane forming in the first portion (17) an interface between a side portion and the portion (41) containing the cells or tissues, said side portion comprising an inlet designed to be supplied with gas by said pressurisation circuit (2) and an outlet for discharging at least a portion of this gas, and **in that** control means (3, 4) are designed to determine the pressure and the time for introducing the gas according to selected criteria, and to control access to the inlet and outlet of the side portion so as to control the shape of the side membrane.

32. A device according to claim 31, **characterized in that** said gas fed into said side portion is the selected gas.

33. A device according to any of claims 1 to 32, **characterized in that** it comprises at least a culture unit (7) including the first tank (8) connected to the culture chamber (6) and a second tank (9) connected to this culture chamber (6) so as to collect the waste culture fluid.

34. A device according to claim 33, **characterized in that** each first (8) and second (9) tank comprises at least a port provided with a leak-proof means (65), in particular of the septum type, designed to allow the introduction or the extraction of fluid.

35. A device according to any of claims 1 to 34, **characterized in that** at least one of the first (8) and second (9) tanks comprises at least a flexible pocket-tank designed to supply with culture fluid said first portion (17) or to collect the waste fluid.

36. A device according to claim 35, **characterized in that** at least the first tank (8) comprises at least two flexible pocket-tanks designed to supply said first portion (17) with different culture fluids.

37. A device according to any of claims 33 to 36, **characterized in that** it comprises a first compartment (1) designed to receive control means (3, 4) and pressurisation circuit (2), and a second compartment (5) designed to receive said culture unit (7).

38. A device according to claim 37, **characterized in that** said second compartment (5) is designed to receive at least another culture unit, and **in that** said pressurisation circuit (2) comprises at least a first bypass portion, connected to each of its outlets (99) delivering said gas under the selected pressure, so as to supply with gas at least the other culture unit.

39. A device according to any of claims 33 to 38, **characterized in that** said pressurisation circuit (2) comprises a second bypass portion (62), connected to each of its outlets (99) delivering said gas under the selected pressure, so as to supply with gas at least an external culture unit.

40. A device according to any of claims 37 to 39, **characterized in that** said first compartment (1) comprises a sub-compartment (25) forming a gas tank supplied with selected gas by each supply inlet (26; 27) and feeding said compressor (30).

41. A device according to claim 40, **characterized in that** said sub-compartment (25) is designed to receive a temperature sensor (34) configured for delivering temperature measurements to the control means (3, 4) and a gas heating means (33) driven by said control means (3, 4) according to said selected criteria and said temperature measurements.

42. A device according to any of claims 40 and 41, **characterized in that** said sub-compartment (25) is designed to receive a gas analyser (35) configured for delivering to the control means (3, 4) representative measurements of the percentage of at least a species of molecules inside said sub-compartment (25), and **in that** said control means (3, 4) are designed to control access to the different inlets and outlets according to said selected criteria and said percentage measurements.

43. A device according to any of claims 40 to 42, combined with any of claims 10 and 12, **characterized in that** said pressurisation circuit (2) includes a duct (100) connecting at least one outlet (74) of said sub-compartment (25) to the inlet (59; 42) of said pocket (43) or of said rear portion (41) of the first portion (17).

44. A device according to any of claims 40 to 43, **characterized in that** said sub-compartment (25) is sub-divided into at least first (67) and second (69) leak-proof sub-portions forming a first low pressure gas tank, supplied with selected gas by each supply inlet (26; 27) and feeding said compressor (30), and a second high pressure gas tank fed by said compressor (30) and supplying each outlet (99, 62) of the pressurisation circuit (2) with selected gas.

45. A device according to claim 44, combined with any of claims 41 and 42, **characterized in that** the first sub-portion (67) of sub-compartment (25) is designed to receive said gas analyser (35) and the second sub-portion (69) of sub-compartment (25) is designed to receive said temperature sensor (34) and said gas heating means (33).

46. A device according to any of claims 44 and 45, **characterized in that** said sub-compartment (25) is sub-divided into first (67), second (69) and third (68) leak-proof sub-portions, said third (68) sub-portion being arranged between the first (67) and second (69) sub-portions and forming a third intermediate pressure gas tank, said sub-portions communicating with one another through a port with access controlled by said control means (3, 4).

47. A device according to any of claims 44 to 46, **characterized in that** each sub-portion (67-69) supplies with selected gas, under control of access control means (49-i) driven by said control means, each outlet (100, 48-i) of the pressurisation circuit, delivering said selected gas to said culture chamber.

48. A method for growing cells and tissues with controlled culture fluid circulation, **characterized in that** it comprises:
- a first step wherein a culture device is provided, comprising at least a culture chamber provided with at least a deformable membrane at the interface between the first and second portions with variable complementary volumes, said first portion being designed to be supplied with a culture fluid and to receive cells and/or tissues to be grown, and said second portion being designed to be supplied with a gas; and
- a second step wherein said first and second portions are fed according to pressures, flow rates and selected times, depending on criteria relating to the type of culture to be achieved, in order to vary the shape of this membrane during the culture period so as to generate a controlled flow of the culture fluid in the first portion of the chamber.

49. A method according to claim 48, **characterized in that** in the first step, a chamber is provided comprising a multiplicity of independent membranes.

50. A method according to claim 49, **characterized in that**, in the first step, a subdivision of the multiplicity into at least two groups of independent membranes is provided, and **in that**, in the second step, each group is independently supplied with selected gas so as to vary the relevant shapes of the membranes of each group, independently.

51. A method according to claim 50, **characterized in that**, in the first step, a subdivision of the multiplicity into at least four groups of independent membranes is provided, and **in that**, in the second step, each group is independently supplied with selected gas so as to vary the relevant shapes of the membranes of each group, independently.

52. A method according to any of claims 49 to 51, **characterized in that**, in the first step, a deformable pocket supplied with gas is provided in the first portion of the culture chamber, on the opposite side to the second part, and **in that**, in the second step, said pocket is fed according to selected pressures, flow rates and times depending on said criteria, in order to vary the shape of this pocket together with that of each membrane during the culture period so as generate culture fluid flow.

53. A method according to any of claims 49 to 51, **characterized in that**, in the first step, at least a deformable auxiliary membrane forming an interface between a central portion receiving the cells or tissues to be grown and a rear portion supplied with gas is provided on the opposite side to the second portion, in the first portion of the culture chamber, and **in that**, in the second step, said rear portion is fed according to pressures, flow rates and times depending on said criteria, in order to vary the shape of the auxiliary membrane together with that of each membrane during the culture period, so to generate culture fluid flow.

54. A method according to any of claims 52 and 53, **characterized in that**, in the first step, the second portion and the pocket or rear portion are supplied with the same selected gas.

55. A method according to any of claims 49 to 54, **characterized in that**, in the first step, every membrane and the pocket or the auxiliary membrane are made of a porous material, at least in the direction pointing towards the first portion, so that the selected gas, fed into the second portion and/or the pocket or the auxiliary membrane, at least partially penetrate said first portion.

56. A method according to any of claims 48 to 55, **characterized in that** the second step is designed to ensure movement of fluid and/or cells and tissues so as to generate in the culture chamber areas where the cell concentrations are different from one another.

57. A method according to claim 56, **characterized in that**, in the second step, differences in cell concentrations are obtained by local changes in the first portion's culture volume.

58. A method according to claim 56, **characterized in that**, in the second step, differences in cell concentrations are obtained through different initial cell concentrations in said culture areas and/or membrane and/or pocket deformation cycles which differ from one area to the other.

59. A method according to any of claims 48 to 58, **characterized in that** the second step is designed to ensure together a control over the pressure and flow of the culture fluid in the first portion of the chamber.

60. A method according to any of claims 53 to 59, **characterized in that**, in the first step, partitioning means are provided in the first portion which define culture cavities, each extending from a group of membranes to a group of auxiliary membranes, and each designed to be supplied with culture fluid and to receive adhering cells as well as culture supports and/or non adhering cells and/or tissues, possibly different from one cavity to another.

## Patentansprüche

1. Vorrichtung für Zell- und Gewebekulturen, mit geregelter Kulturflüssigkeitszirkulation,
**dadurch gekennzeichnet, dass** sie folgendes aufweist: '
- Einen Druckbeaufschlagungskreislauf (2), der in der Lage ist, mindestens ein ausgewähltes Gas mit einem ausgewählten Druck abzugeben,
- mindestens eine Kulturkammer (6), in der Einrichtungen (13) untergebracht sind, die zum Tragen mindestens einer verformbaren Membran (14;46-i) in der Lage sind, die in der Kammer (6) eine Grenzfläche zwischen ersten (17) und zweiten (16) Teilen mit ergänzenden, variablen Volumina bildet, wobei der erste Teil (17) in der Lage ist, zu kultivierende Zellen und/oder Gewebe aufzunehmen und über einen ersten Behälter (8) mit Kulturflüssigkeit versorgt zu werden, und der zweite Teil (16) einen Eingang (18;47-i) aufweist, der in der Lage ist, über den Druckbeaufschlagungskreislauf (2) mit Gas versorgt zu werden, und einen Ausgang (18;47-i) zum Ausstoßen mindestens eines Teils des Gases aufweist, und
- Regelungseinrichtungen (3,4) aufweist, die in der Lage sind, in Abhängigkeit von in Bezug zu der Art der durchzuführenden Kultur stehenden Auswahlkriterien das in den zweiten Teil (16) einzuleitende Gas, dessen Druck und die Zeitdauer der Einleitung des Gases festzulegen, und zum Regeln der Zugänge zu dem ersten (17) und zweiten (16) Teil der Kammer (6) so in der Lage sind, dass diese die Form der Membran (14;46-i), die Versorgung mit Kulturflüssigkeit und die Zirkulation der Flüssigkeit in dem ersten Teil (17) gemeinsam verwalten.

2. Vorrichtung nach Anspruch 1,
**dadurch gekennzeichnet, dass**
sie eine über der Membran (14) auf der Seite des ersten Teils (17) positionierte Über-Membran (90) aufweist, die mit der Membran (14) mindestens eine Zwischenzone (91) abgrenzt, dass die Kammer (6) mindestens einen Eingang (93) aufweist, der in der Lage ist, über den Druckbeaufschlagungskreislauf (2) mit Gas versorgt zu werden und die Zwischenzone (91) mit dem Gas zu versorgen, und mindestens einen Ausgang (94) zum Ausstoßen mindestens eines Teils des Gases aus der Zwischenzone (91) aufweist, und dass die Regelungseinrichtungen (3,4) in der Lage sind, in Abhängigkeit von Auswahlkriterien das in die Zwischenzone (91) einzuleitende Gas, dessen Druck und die Zeitdauer der Einleitung dieses Gases festzulegen, und zum Regeln der Zugänge zu dem Eingang (93) und dem Ausgang (94) der Zwischenzone (91) so in der Lage sind, dass diese die Formen der Membran (14;46-i) und der Über-Membran (90) und die Zirkulation der Kulturflüssigkeit in dem ersten Teil (17) gemeinsam verwalten.

3. Vorrichtung nach Anspruch 2,
**dadurch gekennzeichnet, dass**
die Über-Membran (90) an einer Vielzahl ausgewählter Punkte so an der Membran (14) befestigt ist, dass die Zwischenzone (91) in eine Mehrzahl von miteinander in Verbindung stehenden Zellen (92) mit variablen Volumina unterteilt wird.

4. Vorrichtung nach den Ansprüchen 1 bis 3,
**dadurch gekennzeichnet, dass**
der Druckbeaufschlagungskreislauf (2) mindestens einen mit dem das ausgewählte Gas enthaltenden Behälter verbundenen Eingang und mindestens einen den Eingang (18;47-i) des zweiten Teils (16) versorgenden Ausgang (99) aufweist.

5. Vorrichtung nach einem der Ansprüche 1 bis 4,
**dadurch gekennzeichnet, dass**
der erste Teil (17) mindestens einen Eingang (19) aufweist, der in der Lage ist, über den ersten Behälter (8) mit Kulturflüssigkeit versorgt zu werden, und mindestens einen Ausgang (20) aufweist, der in der Lage ist, die gebrauchte Kulturflüssigkeit in einen zweiten Behälter (9) auszustoßen, und dass die Regelungseinrichtungen (3,4) zum Regeln der Zugänge zu dem verschiedenen Eingängen und Ausgängen des ersten (17) und zweiten (16) Teils der Kammer (6) so in der Lage sind, dass diese die Form der Membran (14;46-i), die Versorgung mit Kulturflüssigkeit und die Zirkulation der Flüssigkeit in dem ersten Teil (17) gemeinsam verwalten.

6. Vorrichtung nach einem der Ansprüche 1 bis 3 in Kombination mit Anspruch 5,
**dadurch gekennzeichnet, dass**
der Druckbeaufschlagungskreislauf (2) von geschlossener Art ist und mindestens einen Eingang (26,27), der in der Lage ist, diesen mit ausgewähltem Gas zu versorgen, einen Kompressor (30), der in der Lage ist, das ausgewählte Gas unter ausgewähltem Druck auszugeben, und mindestens einen von dem Kompressor (30) versorgten Ausgang (99) aufweist, der in der Lage ist, den Eingang (18;47-i) des zweiten Teils (16) zu versorgen.

7. Vorrichtung nach einem der Ansprüche 1 bis 6,
**dadurch gekennzeichnet, dass**
die Trägereinrichtungen (13) in der Lage sind, eine Vielzahl voneinander unabhängiger Membranen (46-i) zu tragen.

8. Vorrichtung nach Anspruch 7,
**dadurch gekennzeichnet, dass**
die Vielzahl in mindestens zwei Gruppen voneinander unabhängiger Membranen unterteilt ist, dass der zweite Teil (16) mindestens zwei Eingänge (47-i) aufweist, um jede Gruppe von Membranen (46-i) unabhängig voneinander mit ausgewähltem Gas zu versorgen, und dass die Regelungseinrichtungen (3,4) zum Regeln der Zugänge zu den zwei Eingängen (48-i) so in der Lage sind, dass diese die jeweiligen Formen der Membranen jeder Gruppe verwalten.

9. Vorrichtung nach Anspruch 7,
**dadurch gekennzeichnet, dass**
die Vielzahl in vier Gruppen voneinander unabhängiger Membranen unterteilt ist, dass der zweite Teil (16) mindestens vier Eingänge (47-i) aufweist, um jede Gruppe von Membranen unabhängig voneinander mit ausgewähltem Gas zu versorgen, und dass die Regelungseinrichtungen (3,4) zum Regeln der Zugänge zu den vier Eingängen so in der Lage sind, dass diese die jeweiligen Formen der Membranen jeder Gruppe (46-i)verwalten.

10. Vorrichtung nach einem der Ansprüche 1 bis 9,
**dadurch gekennzeichnet, dass**
in der Kulturkammer (6) in dem ersten Teil (17) auf der dem zweiten Teil (16) gegenüberliegenden Seite ein verformbarer Beutel (43) untergebracht ist, der mit einem durch den Druckbeaufschlagungskreislauf (2) mit Gas versorgten Eingang (59) und mit einem mit dem Druckbeaufschlagungskreislauf (2) verbundenen Ausgang (59) ausgestattet ist, und dass die Regelungseinrichtungen (3,4) in der Lage sind, in Abhängigkeit von in Bezug zu der Art der durchzuführenden Kultur stehenden Auswahlkriterien den Druck und die Zeitdauer der Einleitung des Gases festzulegen, und zum Regeln der Zugänge zu dem Eingang und zu dem Ausgang des Beutels (43) so in der Lage sind, dass diese dessen Form gemeinsam mit der Form jeder Membran (46-i) verwalten.

11. Vorrichtung nach Anspruch 10,
**dadurch gekennzeichnet, dass**
der Beutel (43) aus einer Membran gebildet ist.

12. Vorrichtung nach einem der Ansprüche 1 bis 9,
**dadurch gekennzeichnet, dass**
in der Kulturkammer (6) in dem ersten Teil (17) auf der dem zweiten Teil (16) gegenüberliegenden Seite Hilfsträgereinrichtungen (12) untergebracht sind, die in der Lage sind, mindestens eine verformbare Hilfsmembran (39) zu tragen, die in dem ersten Teil (17) eine Grenzfläche zwischen einem mittleren Teil (40), der in der Lage ist, zu kultivierende Zellen oder Gewebe aufzunehmen, und einem hinteren Teil (41) mit einem Eingang (42) bildet, der in der Lage ist, über den Druckbeaufschlagungskreislauf (2) mit Gas versorgt zu werden, und einen mit dem Druckbeaufschlagungskreislauf (2) verbundenen Ausgang (42) aufweist, und dass die Regelungseinrichtungen (3,4) in der Lage sind, in Abhängigkeit von in Bezug zu der Art der durchzuführenden Kultur stehenden Auswahlkriterien den Druck und die Zeitdauer der Einleitung des Gases festzulegen, und zum Regeln der Zugänge zu dem Eingang und zu dem Ausgang des hinteren Teils (41) so in der Lage sind, dass diese die Form der Hilfsmembran (39) gemeinsam mit der Form jeder Membran (14;46-i) verwalten.

13. Vorrichtung nach einem der Ansprüche 10 bis 12,
**dadurch gekennzeichnet, dass**
der Druckbeaufschlagungskreislauf (2) einen weiteren Ausgang (100) aufweist, der in der Lage ist, den Eingang (59;42) des Beutels (43) oder des hinteren Teils (41) mit dem ausgewählten Gas mit einem anderen, ausgewählten Druck zu versorgen.

14. Vorrichtung nach einem der Ansprüche 10 bis 13,
**dadurch gekennzeichnet, dass**
der Eingang und der Ausgang des Beutels (43) oder des hinteren Teils (41) ineinander übergehen.

15. Vorrichtung nach einem der Ansprüche 10 bis 14,
**dadurch gekennzeichnet, dass**
die Hilfsträgereinrichtungen (12) zum Tragen einer Vielzahl voneinander unabhängiger Hilfsmembranen in der Lage sind.

16. Vorrichtung nach Anspruch 15,
**dadurch gekennzeichnet, dass**
die Vielzahl in mindestens zwei Gruppen voneinander unabhängiger Hilfsmembranen unterteilt ist, dass der hintere Teil (41) mindestens zwei Eingänge aufweist, um jede Gruppe von Hilfsmembranen unabhängig voneinander mit ausgewähltem Gas zu versorgen, und dass die Regelungseinrichtungen (3,4) zum Regeln der Zugänge zu den vier Eingängen so in der Lage sind, dass diese die jeweiligen Formen der Hilfsmembranen jeder Gruppe verwalten.

17. Vorrichtung nach Anspruch 15,
**dadurch gekennzeichnet, dass**
die Vielzahl in vier Gruppen voneinander unabhängiger Hilfsmembranen unterteilt ist, dass der hintere Teil (41) vier Eingänge aufweist, um jede Gruppe von Hilfsmembranen unabhängig voneinander mit ausgewähltem Gas zu versorgen, und dass die Regelungseinrichtungen (3,4) zum Regeln der Zugänge zu den vier Eingängen so in der Lage sind, dass diese die jeweiligen Formen der Hilfsmembranen jeder Gruppe verwalten. '

18. Vorrichtung nach einem der Ansprüche 8 und 9 in Kombination mit einem der Ansprüche 16 und 17,
**dadurch gekennzeichnet, dass**
in dem mittleren Teil (40) des ersten Teils (17) Unterteilungseinrichtungen untergebracht sind, die innerhalb desselben Kulturvertiefungen festlegen, die sich jeweils von einer Gruppe von Membranen (46-i) zu einer Gruppe von Hilfsmembranen erstrecken, wobei jede Vertiefung in der Lage ist, mit Kulturflüssigkeit versorgt zu werden.

19. Vorrichtung nach Anspruch 18,
**dadurch gekennzeichnet, dass**
die Unterteilungseinrichtungen aus einem für die Kulturflüssigkeit porösen, jedoch für Zellen und Gewebe undurchlässigen Werkstoff ausgeführte Trennwände aufweisen, so dass die Kulturflüssigkeit von einer Vertiefung zur anderen zirkulieren kann.

20. Vorrichtung nach einem der Ansprüche 1 bis 19,
**dadurch gekennzeichnet, dass**
der erste Teil (17) mindestens eine mit einer dichten Einrichtung, insbesondere einer Art Septum, ausgestattete Öffnung aufweist, die in der Lage ist, die Einleitung oder die Extraktion von Zellen oder Geweben zu ermöglichen.

21. Vorrichtung nach einem der Ansprüche 1 bis 20,
**dadurch gekennzeichnet, dass**
der Eingang und der Ausgang (18;47-i) des zweiten Teils (16) ineinander übergehen.

22. Vorrichtung nach einem der Ansprüche 1 bis 21,
**dadurch gekennzeichnet, dass**
die Membranen (14;39;43;46-i) und Hilfsmembranen mindestens in Richtung des ersten Teils (17) aus einem porösen Werkstoff ausgeführt sind, so dass das in den zweiten Teil (16) eingeleitete, ausgewählte Gas mindestens teilweise in den ersten Teil (17) eindringen kann.

23. Vorrichtung nach einem der Ansprüche 5 bis 22,
**dadurch gekennzeichnet, dass**
die Kulturkammer (6) durch ein Gehäuse begrenzt wird, welches einen unteren Teil aufweist, der einen Auffangbehälter (11) bildet, der mindestens eine an den Eingang (19) zur Versorgung mit Kulturflüssigkeit angepasste Öffnung aufweist, wobei der Ausgang (20) zum Ausstoßen der gebrauchten Kulturflüssigkeit, der Eingang (18;46-i) (oder die Eingänge) des zweiten Teils (16) zur Versorgung mit ausgewähltem Gas und der Eingang (oder die Eingänge) (18;22) mit dem Druckbeaufschlagungskreislauf (2) verbunden sind, wobei ein oberer Teil einen Deckel (12) bildet, und dass die Trägereinrichtungen ein in dem Auffangbehälter (11) untergebrachtes Tragelement (44) umfassen, in dem die Vertiefungen (45-i) festgelegt sind, die jeweils in der Lage sind, abgedichtet eine Membran (46-i) zu tragen, mit dem ausgewählten Gas versorgt zu werden und die Membran aufzunehmen, wenn der Druck des ausgewählten Gases unter einem ersten Schwellenwert liegt.

24. Vorrichtung nach Anspruch 23,
**dadurch gekennzeichnet, dass**
der Deckel (12) aus einem durchsichtigen Werkstoff ausgeführt ist.

25. Vorrichtung nach einem der Ansprüche 23 und 24,
**dadurch gekennzeichnet, dass**
der Auffangbehälter (11) eine Öffnung für jeden Eingang und für jeden Ausgang des ersten (17) und zweiten (16) Teils der Kammer (6) aufweist.

26. Vorrichtung nach einem der Ansprüche 23 und 24 in Kombination mit einem der Ansprüche 10 und 12,
**dadurch gekennzeichne**t, dass
der Auffangbehälter (11) eine Öffnung für jeden Eingang und für jeden Ausgang des zweiten Teils (16) der Kammer (6) aufweist, dass der Deckel (12) eine an den Eingang und an den Ausgang (42;59) des Beutels (43) oder des hinteren Teils (41) angepasste Öffnung aufweist, und dass die Hilfsträgereinrichtungen fest mit dem Deckel (12) verbunden sind.

27. Vorrichtung nach einem der Ansprüche 23 bis 26,
**dadurch gekennzeichnet, dass**
sie eine mindestens aus einer pH-Sonde (37) und einer Temperatursonde (36) ausgewählte Sonde umfasst, die mit den Regelungseinrichtungen (3) verbunden und in der Lage ist, in das Innere des ersten Teils (17) der Kammer (6) eingeführt zu werden, und dass der Auffangbehälter (11) mindestens eine zur Einführung jeder Sonde (36;38) angepasste Hilfsöffnung (52,51) aufweist.

28. Vorrichtung nach einem der Ansprüche 1 bis 27,
**dadurch gekennzeichnet, dass**
sie zwei von den Regelungseinrichtungen (3,4) gesteuerte Eingänge (26,27) aufweist, die dazu in der Lage sind, jeweils mit erstem und zweitem ausgewähltem Gas versorgt zu werden.

29. Vorrichtung nach einem der Ansprüche 1 bis 28,
**dadurch gekennzeichnet, dass** jeder Eingang und jeder Ausgang durch eine von den Regelungseinrichtungen (3,4) gesteuerte Zugangsregelungseinrichtung (28;29;32;49-i;75) gesteuert wird.

30. Vorrichtung nach einem der Ansprüche 23 bis 29,
**dadurch gekennzeichnet, dass**
das Tragelement (44) aus einem synthetischen Werkstoff, insbesondere aus einem Elastomer, ausgeführt ist.

31. Vorrichtung nach einem der Ansprüche 1 bis 30,
**dadurch gekennzeichnet, dass**
in dem ersten Teil (17) mindestens eine verformbare, seitliche Membran untergebracht ist, die in dem ersten Teil (17) eine Grenzfläche zwischen einem seitlichen Teil und dem die Zellen oder Gewebe enthaltenden Teil (41) bildet, wobei der seitliche Teil einen Eingang aufweist, der in der Lage ist, über den Druckbeaufschlagungskreislauf (2) mit Gas versorgt zu werden, und einen Ausgang zum Ausstoßen mindestens eines Teils des Gases aufweist, und dass die Regelungseinrichtungen (3,4) in der Lage sind, in Abhängigkeit von Auswahlkriterien den Druck und die Zeitdauer der Einleitung des Gases festzulegen, und zum Regeln der Zugänge zu dem Eingang und dem Ausgang des seitlichen Teils so in der Lage sind, dass diese die Form der seitlichen Membran gemeinsam verwalten.

32. Vorrichtung nach Anspruch 31,
**dadurch gekennzeichnet, dass**
es sich bei dem in den seitlichen Teil eingeleitete Gas um das ausgewählte Gas handelt.

33. Vorrichtung nach einem der Ansprüche 1 bis 32,
**dadurch gekennzeichnet, dass**
sie mindestens eine Kultureinheit (7) aufweist, die den mit der Kulturkammer (6) verbundenen, ersten Behälter (8), und einen zweiten Behälter (9) aufweist, der so mit der Kulturkammer (6) verbunden ist, dass er die gebrauchte Kulturflüssigkeit sammelt.

34. Vorrichtung nach Anspruch 33,
**dadurch gekennzeichnet, dass**
jeder erste (8) und zweite (9) Behälter mindestens eine mit einer dichten Einrichtung (65), insbesondere einer Art Septum, ausgestattete Öffnung aufweist, die in der Lage ist, die Einleitung oder die Extraktion von Flüssigkeit zu ermöglichen.

35. Vorrichtung nach einem der Ansprüche 1 bis 34,
**dadurch gekennzeichnet, dass**
mindestens einer aus den ersten (8) und zweiten (9) Behältern mindestens einen nachgiebigen, beutelförmigen Behälter aufweist, der in der Lage ist, den ersten Teil (17) mit Kulturflüssigkeit zu versorgen oder die gebrauchte Flüssigkeit zu sammeln.

36. Vorrichtung nach Anspruch 35,
**dadurch gekennzeichnet, dass**
mindestens der erste Behälter (8) mindestens zwei nachgiebige, beutelförmige Behälter aufweist, die in der Lage sind, den ersten Teil (17) mit verschiedenen Kulturflüssigkeiten zu versorgen.

37. Vorrichtung nach einem der Ansprüche 33 bis 36,
**dadurch gekennzeichnet, dass**
sie ein erstes Abteil (1), in dem die Regelungseinrichtungen (3,4) und der Druckbeaufschlagungskreislauf (2) untergebracht sind, und ein zweites Abteil (5) aufweist, in dem die Kultureinheit (7) untergebracht ist.

38. Vorrichtung nach Anspruch 37,
**dadurch gekennzeichnet, dass**
in dem zweiten Abteil (5) mindestens eine weitere Kultureinheit untergebracht ist, und dass der Druckbeaufschlagungskreislauf (2) mindestens einen ersten Umleitungsteil aufweist, der mit jedem seiner das Gas mit einem ausgewählten Druck abgebenden Ausgänge (99) so verbunden ist, dass er mindestens die weitere Kultureinheit mit Gas versorgt.

39. Vorrichtung nach einem der Ansprüche 33 bis 38,
**dadurch gekennzeichnet, dass**
der Druckbeaufschlagungskreislauf (2) einen zweiten Umleitungsteil (62) aufweist, der mit jedem seiner das Gas mit einem ausgewählten Druck abgebenden Ausgänge (99) so verbunden ist, dass er mindestens eine Kultureinheit mit Gas versorgt.

40. Vorrichtung nach einem der Ansprüche 37 bis 39,
**dadurch gekennzeichnet, dass**
das erste Abteil (1) ein Unterabteil (25) aufweist, welches einen von jedem Versorgungseingang (26;27) mit ausgewähltem Gas' versorgten Behälter bildet und den Kompressor (30) versorgt.

41. Vorrichtung nach Anspruch 40,
**dadurch gekennzeichnet, dass**
in dem Unterabteil (25) ein Temperatursensor (34), der in der Lage ist, die Temperaturmessungen an die Regelungseinrichtungen (3,4) auszugeben, und eine von den Regelungseinrichtungen (3,4) in Abhängigkeit von den ausgewählten Kriterien und den Temperaturmessungen gesteuerte Gasheizeinrichtung (33) untergebracht sind.

42. Vorrichtung nach einem der Ansprüche 40 und 41,
**dadurch gekennzeichnet, dass**
in dem Unterabteil (25) ein Gasanalysator (35) untergebracht ist, der in der Lage ist, den Regelungseinrichtungen (3,4) repräsentative Messungen des Prozentsatzes mindestens einer Molekülart im Inneren des Unterabteils (25) zu liefern, und dass die Regelungseinrichtungen (3,4) in der Lage sind, den Zugang zu den verschiedenen Eingängen und Ausgängen in Abhängigkeit von den ausgewählten Kriterien und von den Prozentsatzmessungen zu steuern.

43. Vorrichtung nach einem der Ansprüche 40 bis 42 in Kombination mit einem der Ansprüche 10 und 12,
**dadurch gekennzeichnet, dass**
der Druckbeaufschlagungskreislauf (2) einen Kanal (100) aufweist, der mindestens einen Ausgang des Unterabteils (25) mit dem Eingang (59;42) des Beutels (43) oder des hinteren Teils (41) des ersten Teils (17) verbindet.

44. Vorrichtung nach einem der Ansprüche 40 bis 43,
**dadurch gekennzeichnet, dass**
das Unterabteil (25) in mindestens erste (67) und zweite (69) dichte Unter-Teile unterteilt ist, die jeweils einen ersten Niederdruckgasbehälter, der von jedem Vorsorgungseingang (26;27) mit ausgewähltem Gas versorgt wird und den Kompressor (30) versorgt, und einen zweiten, von dem Kompressor (30) versorgten Hochdruckgasbehälter bilden, der jeden Ausgang (99,62) des Druckbeaufschlagungskreislaufes (2) mit ausgewähltem Gas versorgt.

45. Vorrichtung nach Anspruch 44 in Kombination mit den Ansprüchen 41 und 42,
**dadurch gekennzeichnet, dass**
in dem ersten Unter-Teil (67) des Unterabteils (25) der Gasanalysator (35), und in dem zweiten Unter-Teil (69) des Unterabteils (25) der Temperatursensor (34) und die Gasheizeinrichtung (33) untergebracht sind.

46. Vorrichtung nach einem der Ansprüche 44 und 45,
**dadurch gekennzeichnet, dass**
das Unterabteil (25) in erste (67), zweite (69) und dritte (68) dichte Unter-Teile unterteilt ist, wobei der dritte Unter-Teil (68) zwischen den ersten (67) und zweiten (69) Unter-Teilen positioniert ist einen dritten Behälter für Gas mit mittlerem Druck bilden, wobei die Unter-Teile durch eine Öffnung mit von den Regelungseinrichtungen (3,4) gesteuertem Zugang miteinander verbunden sind.

47. Vorrichtung nach einem der Ansprüche 44 bis 46,
**dadurch gekennzeichnet, dass**
jeder Unter-Teil (67-69) jeden Ausgang (100,48-i) des Druckbeaufschlagungskreislaufes, der das ausgewählte Gas an die Kulturkammer ausgibt, unter der Regelung der von Regelungseinrichtungen gesteuerten Zugangsregelungseinrichtungen (49-i) mit ausgewähltem Gas versorgt.

48. Verfahren für Zell- und Gewebekulturen, mit geregelter Kulturflüssigkeitszirkulation,
**dadurch gekennzeichnet, dass** es folgendes umfasst:
- Eine erste Etappe, in der eine Kulturvorrichtung vorgesehen ist, die mindestens eine Kulturkammer aufweist, die mit mindestens einer verformbaren Membran an der Grenzfläche zwischen ersten und zweiten Teilen mit ergänzenden, variablen Volumina ausgestattet ist, wobei der erste Teil in der Lage ist, mit einer Kulturflüssigkeit versorgt zu werden und zu kultivierende Zellen und/oder Gewebe aufzunehmen, und der zweite Teil in der Lage ist, mit einem Gas versorgt zu werden; und
- eine zweite Etappe, in der die ersten und zweiten Teile gemäß den in Abhängigkeit von in Bezug zu der Art der durchzuführenden Kultur stehenden Auswahlkriterien ausgewählten Drücken, Durchsätzen und Zeitdauern versorgt werden, um die Form der Membran während der Zeitdauer der Kultur zur Veränderung zu veranlassen, so dass eine kontrollierte Zirkulation der Kulturflüssigkeit in dem ersten Teil der Kammer erzeugt wird.

49. Verfahren nach Anspruch 48,
**dadurch gekennzeichnet, dass**
bei der ersten Etappe eine Kammer vorgesehen ist, die eine Vielzahl voneinander unabhängiger Membranen aufweist.

50. Verfahren nach Anspruch 49,
**dadurch gekennzeichnet, dass**
bei der ersten Etappe eine Unterteilung der Vielzahl in mindestens zwei Gruppen voneinander unabhängiger Membranen vorgesehen ist, und dass bei der zweiten Etappe jede Gruppe unabhängig mit ausgewähltem Gas versorgt wird, so dass die jeweiligen Formen der Membranen jeder Gruppe unabhängig voneinander zur Veränderung veranlasst werden.

51. Verfahren nach Anspruch 50,
**dadurch gekennzeichnet, dass**
bei der ersten Etappe eine Unterteilung der Vielzahl in mindestens vier Gruppen voneinander unabhängiger Membranen vorgesehen ist, und dass bei der zweiten Etappe jede Gruppe unabhängig mit ausgewähltem Gas versorgt wird, so dass die jeweiligen Formen der Membranen jeder Gruppe unabhängig voneinander zur Veränderung veranlasst werden.

52. Verfahren nach einem der Ansprüche 49 bis 51,
**dadurch gekennzeichnet, dass**
bei der ersten Etappe in dem ersten Teil der Kulturkammer auf der dem zweiten Teil gegenüberliegenden Seite ein mit Gas versorgter, verformbarer Beutel vorgesehen ist, und dass der Beutel bei der zweiten Etappe gemäß den in Abhängigkeit von in Bezug zu der Art der durchzuführenden Kultur stehenden Auswahlkriterien ausgewählten Drücken, Durchsätzen und Zeitdauern versorgt wird, um die Form des Beutels gemeinsam mit derjenigen einer jeden Membran während der Zeitdauer der Kultur zur Veränderung zu veranlassen, so dass die Zirkulation der Kulturflüssigkeit erzeugt wird.

53. Verfahren nach einem der Ansprüche 49 bis 51,
**dadurch gekennzeichnet, dass**
bei der ersten Etappe in dem ersten Teil der Kulturkammer auf der dem zweiten Teil gegenüberliegenden Seite mindestens eine verformbare Hilfsmembran vorgesehen ist, welche die Grenzfläche zwischen einem mittleren Teil, der zu kultivierende Zellen oder Gewebe aufnimmt, und einem mit Gas versorgten, hinteren Teil bildet, und dass der hintere Teil bei der zweiten Etappe gemäß den in Abhängigkeit von in Bezug zu der Art der durchzuführenden Kultur stehenden Auswahlkriterien ausgewählten Drücken, Durchsätzen und Zeitdauern versorgt wird, um die Form der Hilfsmembran gemeinsam mit derjenigen einer jeden Membran während der Zeitdauer der Kultur zur Veränderung zu veranlassen, so dass die Zirkulation der Kulturflüssigkeit erzeugt wird.

54. Verfahren nach einem der Ansprüche 52 und 53,
**dadurch gekennzeichnet, dass**
bei der ersten Etappe der zweite Teil und der Beutel oder der hintere Teil mit demselben ausgewählten Gas versorgt werden.

55. Verfahren nach einem der Ansprüche 49 bis 54,
**dadurch gekennzeichnet, dass**
bei der ersten Etappe jede Membran oder die Hilfsmem-bran mindestens in Richtung des ersten Teils aus einem porösen Werkstoff ausgeführt sind, so dass das in den zweiten Teil und/oder den Beutel oder die Hilfsmembran eingeleitete, ausgewählte Gas mindestens teilweise in den ersten Teil eindringen kann.

56. Verfahren nach einem der Ansprüche 48 bis 55,
**dadurch gekennzeichnet, dass**
die zweite Etappe in der Lage ist, eine Verlagerung der Flüssigkeit und/oder der Zellen und Gewebe sicherzustellen, so dass in der Kulturkammer Zonen erzeugt werden, in welchen die Zellkonzentrationen untereinander unterschiedlich sind.

57. Verfahren nach Anspruch 56,
**dadurch gekennzeichnet, dass**
bei der zweiten Etappe die Unterschiede bei den Zellkonzentrationen durch örtliche Veränderungen des Kulturvolumens des ersten Teils bewirkt werden.

58. Verfahren nach Anspruch 56,
**dadurch gekennzeichnet, dass**
bei der zweiten Etappe die Unterschiede bei den Zellkonzentrationen durch in den Kulturzonen unterschiedliche Anfangskonzentrationen und/oder durch von einer zur anderen Zone unterschiedliche Verformungszyklen der Membran(en) und/oder Beutel erhalten werden.

59. Verfahren nach einem der Ansprüche 48 bis 58,
**dadurch gekennzeichnet, dass**
die zweite Etappe in der Lage ist, gleichzeitig eine Druck- und Zirkulationssteuerung der Kulturflüssigkeit in dem ersten Teil der Kammer sicherzustellen.

60. Verfahren nach einem der Ansprüche 53 bis 59,
**dadurch gekennzeichnet, dass**
bei der ersten Etappe in dem ersten Teil Unterteilungseinrichtungen vorgesehen sind, die innerhalb desselben Kulturvertiefungen festlegen, die sich jeweils von einer Gruppe von Membranen zu einer Gruppe von Hilfsmembranen erstrecken, die jeweils dazu in der Lage sind, mit Kulturflüssigkeit versorgt zu werden und haftende Zellen sowie Kulturträger und/oder nicht haftende Zellen und/oder Gewebe aufzunehmen, die möglicherweise von einer Vertiefung zu einer anderen unterschiedlich sind.
